# EUROPEAN PATENT APPLICATION

(11) **EP 3 901 168 A1**
(43) Date of publication of application: **27.10.2021**
(21) Application number: 20170651.2
(22) Date of filing: 21.04.2020
(51) Int. Cl.: C07K 14/56, A61P 31/12

(54) **INTERFERON ALPHA 2 VARIANTS AND USES THEREOF**

(71) Applicant: Universität Duisburg-Essen, 45141 Essen (DE)
(72) Inventor: DITTMER, Ulf, 45219 Essen (DE); SUTTER, Kathrin, 45472 Mülheim an der Ruhr (DE); TRILLING, Mirko, 45131 Essen (DE)
(74) Representative: Viering, Jentschura & Partner mbB Patent- und Rechtsanwälte

(57) **Abstract**

The present invention relates to recombinant interferon alpha 2 (IFNa2) mutants and variants as well as fragments thereof, nucleic acids encoding them, pharmaceutical compositions containing them, and methods of use thereof.

## Description

### FIELD OF THE INVENTION

The present invention lies in the field of molecular biology and recombinant/chimeric polypeptides/proteins and relates to recombinant interferon alpha 2 (IFNa2) mutants and variants as well as fragments thereof, pharmaceutical compositions containing them, and methods of use thereof.

### BACKGROUND OF THE INVENTION

Interferons (IFNs) are a family of proteins that were originally named for their ability to interfere with viral replication and propagation. To date, it is known that interferons are also involved in combating bacterial and parasitic infections, inhibit cell division, and promote or impede the differentiation of cells. The interferons are classified based on their receptor specificity into three types of interferons: type I, type II and type III. The interferons of type I are monomeric proteins and include IFN alpha, IFN beta and IFN omega that are products of leukocytes and fibroblasts, IFN kappa that is expressed by human keratinocytes, IFN epsilon that is exclusively expressed in lung, brain, small intestine and the reproductive tissue and IFN tau that has been described only in ruminants. The only known type II interferon is the dimeric IFN gamma, which is produced exclusively by lymphocytes. The interferons of type III are monomeric proteins and include IFN lambda1 (IL29), IFN lambda 2 (IL28A), IFN lambda 3 (IL28B), and IFN lambda 4 and signal only on epithelial cells.

The interferon alpha family is composed of 13 intron-less fully translated genes (excluding pseudogenes). Each member includes mature proteins of 165 or 166 amino acid residues, with two conserved disulfide bonds: Cys1-Cys98 and Cys29-Cys138. A high level of sequence homology (70-99%) is displayed among the various interferon alpha subtypes, and about 35% homology exists between these subtypes and IFN beta. Despite the high homology, a shared 3D core structure and a shared receptor of the different subtypes, their biological activities, among them anti-proliferative, antiviral, and immunomodulation, differ notably.

While they are highly active antiviral agents, they are not widely used for the treatment of viral infections. Of the known IFN alpha subtypes, only interferon alpha 2 (IFNa2) has been extensively studied for its pharmaceutical potential. IFNa2 is therapeutically used for treatment of chronic HBV- or HBV/HDV-co-infection, but leads to a sustained virus control in less than 20% of the treated patients. In chronic HIV infection its efficacy is even lower, with the results that it has been clinically used in only very few studies.

IFNa2 is also known to have anti-cancer effects. Here it is mainly used in second line adjunct therapy of hematopoietic cancers. However, this treatment is not always effective and sometimes results in intolerable side effects related to the dosage and duration of therapy.

Worldwide there are nearly 300 million people infected with HIV or HBV which are in need of more efficient therapies.

Other dangerous virus infections, such as influenza or corona virus infections, have not yet been treated with IFNa2, since its activity in virus control was found to be too low for efficient treatments. Accordingly, the available treatments for infectious diseases are effective only in a certain percentage of patients, are expensive and adverse side effects are not uncommon. There thus remains an unmet medical need for suitable therapeutic methods that are safe, reliable, efficacious, and cost effective.

It has been found that other interferon alpha subtypes, such as IFNa14 and IFNa16, but also IFNa6, have a much higher potential for treatment of certain viral infections, such as HIV and influenza virus infection (Lavender et al. (2016) J. Virol. 90(13):6001-13; Sutter et al. (2019) Sci Rep. 9(1):18089; Harper et al. (2015) PLoS Pathog. 11(11):e1005254; da Rocha Matos et al. (2019), Emerging Microbes & Infection, Vol. 8:1763-76). However, these interferon alpha subtypes have not been tested in clinical studies yet, as high costs for the development of drugs based on these proteins are prohibitive.

### SUMMARY OF THE INVENTION

The present invention is based on the inventors' surprising finding that IFNa14 is more efficient for the treatment of HBV infections. Based on these and earlier findings regarding the different antiviral activities of interferon alpha subtypes, the inventors designed chimeric mutants of the known IFNa2 and IFNa6/IFNa14/IFNa16 proteins that use the IFNa2 backbone, which is clinically well-established, with a variety of point mutations derived from the IFNa6/IFNa14/IFNa16 amino acid sequences that show significantly higher antiviral activity than IFNa2.

In a first aspect, the present invention therefore relates to a recombinant interferon alpha 2 (IFNa2) polypeptide comprising an amino acid sequence having at least 80 %, at least 85 %, at least 90 %, at least 95%, at least 97.5%, at least 99% or at least 99.5% sequence identity over its entire length with the amino acid sequence set forth in SEQ ID NO:1 and comprising one or more amino acid substitution(s) in any one or more of the positions corresponding to positions 2, 10, 11, 14, 15, 16, 22, 26, 34, 37, 42, 51, 52, 54, 55, 56, 59, 60, 63, 74, 79, 82, 84, 86, 89, 92, 100, 102, 104, 106, 107, 108, 109, 112, 120, 131, 132, 153, 155, 159, 160, 163, and 165 of SEQ ID NO:1, or fragments thereof. Said recombinant IFNa2 polypeptide, typically derived from SEQ ID NO:1 by one or more amino acid substitution(s), is also referred to herein as "IFNa2 variant" or "IFNa2 chimeric protein" or "IFNa2 mutant".

In various embodiments, the recombinant IFNa2 polypeptide of the invention comprises amino acid substitution(s) in
(i) any one or more of the positions corresponding to positions 2, 10, 26, 37, 51, 52, 54, 60, 63, 82, 86, 89, 92, 120, 131, and 153 of SEQ ID NO:1;
(ii) any one or more of the positions corresponding to positions 11, 14, 16, 22, 26, 34, 42, 51, 52, 54, 55, 56, 63, 86, 89, 100, 102, 106, 108, 109, 112, 131, 132, 153, 159, 160, 163, and 165 of SEQ ID NO:1;
(iii) any one or more of the positions corresponding to positions 11, 15, 37, 52, 54, 59, 63, 74, 79, 84, 100, 102, 104, 106, 107, 112, 131, 153, 155, 160, and 163 of SEQ ID NO:1;
(iv) the positions corresponding to positions 82, 86, 89, and 120 of SEQ ID NO:1;
(v) the positions corresponding to 26 and 153 of SEQ ID NO:1;
(vi) the positions corresponding to positions 26, 82, 86, 89, 120, and 153 of SEQ ID NO:1;
(vii) the positions corresponding to positions 51, 52, 54, 60, and 63 of SEQ ID NO:1;
(viii) the positions corresponding to positions 2, 10, 37, 92, and 131 of SEQ ID NO:1;
(ix) the positions corresponding to positions 86 and 89 of SEQ ID NO:1;
(x) the positions corresponding to positions 16, 22, 26, and 153 of SEQ ID NO:1;
(xi) the positions corresponding to positions 16, 22, 26, 86, 89, and 153 of SEQ ID NO:1;
(xii) the positions corresponding to positions 52, 54, 55, 56, and 63 of SEQ ID NO:1;
(xiii) the positions corresponding to positions 11, 14, 34, 42, 51, 100, 102, 106, 108, 109, 112, 131, 132, 159, 160, 163, and 165 of SEQ ID NO:1;
(xiv) the positions corresponding to positions 15 and 153 of SEQ ID NO:1;
(xv) the positions corresponding to positions 52, 54, 59, and 63 of SEQ ID NO:1; or
(xvi) the positions corresponding to positions 11, 37, 74, 79, 84, 100, 102, 104, 106, 107, 112, 131, 155, 160 and 163 of SEQ ID NO:1.

In any of the embodiments described herein, the amino acid substitution(s) in the above-listed positions may, without limitation, be selected from the group consisting of: 2N, 10N, 11N, 11H, 14A, 15M, 161, 22G, 26P, 26Y, 34N, 37E, 37R, 42V, 51Q, 52A, 54S, 55A, 56F, 59V, 60M, 63T, 74V, 79R, 82E, 84L, 86I, 89F, 92M, 100T, 100M, 102E, 104W, 106G, 106E, 107G, 1081, 109A, 112N, 120K, 131M, 131T, 132G, 153F, 153S, 155R, 159K, 160G, 160R, 163R, and 165D using the positional numbering of SEQ ID NO:1.

Accordingly, in various embodiments, the recombinant IFNa2 polypeptide comprises, using the numbering of SEQ ID NO:1,
(i) any one or more amino acid substitution(s) selected from the group consisting of 2N, 10N, 26P, 37E, 51Q, 52A, 54S, 60M, 63T, 82E, 86I, 89F, 92M, 120K, 131M, and 153F;
(ii) any one or more amino acid substitution(s) selected from the group consisting of 11N, 14A, 161, 22G, 26Y, 34N, 42V, 51Q, 52A, 54S, 55A, 56F, 63T, 86I, 89F, 100T, 102E, 106E, 1081, 109A, 112N, 131M, 132G, 153F, 159K, 160G, 163R, and 165D;
(iii) any one or more of the positions corresponding to positions 11H, 15M, 37R, 52A, 54S, 59V, 63T, 74V, 79R, 84L, 100M, 102E, 104W, 106G, 107G, 112N, 131T, 153S, 155R, 160R and 163R of SEQ ID NO:1;
(iv) the amino acid substitution(s) 82E, 86I, 89F, and 120K;
(v) the amino acid substitution(s) 26P and 153F;
(vi) the amino acid substitution(s) 26P, 82E, 86I, 89F, 120K, and 153F;
(vii) the amino acid substitution(s) 51Q, 52A, 54S, 60M, and 63T;
(viii) the amino acid substitution(s) 2N, 10N, 37E, 92M, and 131M;
(ix) the amino acid substitution(s) 861 and 89F;
(x) the amino acid substitution(s) 161, 22G, 26Y, and 153F;
(xi) the amino acid substitution(s) 161, 22G, 26Y, 861, 89F, and 153F;
(xii) the amino acid substitution(s) 52A, 54S, 55A, 56F, and 63T;
(xiii) the amino acid substitution(s) 11N, 14A, 34N, 42V, 51Q, 100T, 102E, 106E, 1081, 109A, 112N, 131M, 132G, 159K, 160G, 163R, and 165D;
(xiv) the positions corresponding to positions 15M and 153S of SEQ ID NO:1;
(xv) the positions corresponding to positions 52A, 54S, 59V, and 63T of SEQ ID NO:1; or
(xvi) the positions corresponding to positions 11H, 37R, 74V, 79R, 84L, 100M, 102E, 104W, 106G, 107G, 112N, 131T, 155R, 160R, and 163R of SEQ ID NO:1.

In various embodiments, the sets of substitutions defined above as (iv)-(xvi) may also be combined. In such instances, the recombinant IFNa2 polypeptide may comprise, using the numbering of SEQ ID NO:1,
(A) any combination of two or more of the following sets of amino acid substitution(s)
   (1) 82E, 86I, 89F, and 120K;
   (2) 26P and 153F;
   (3) 51Q, 52A, 54S, 60M, and 63T;
   (4) 2N, 10N, 37E, 92M, and 131M;
(B) any combination of two or more of the following sets of amino acid substitution(s)
   (1) 861 and 89F;
   (2) 161, 22G, 26Y, and 153F;
   (3) 52A, 54S, 55A, 56F, and 63T;
   (4) 11N, 14A, 34N, 42V, 51Q, 100T, 102E, 106E, 1081, 109A, 112N, 131M, 132G, 159K, 160G, 163R, and 165D;
(C) any combination of two or more of the following sets of amino acid substitution(s)
   (1) 15M and 153S;
   (2) 52A, 54S, 59V, and 63T;
   (3) 11H, 37R, 74V, 79R, 84L, 100M, 102E, 104W, 106G, 107G, 112N, 131T, 155R, 160R, and 163R.

Concrete combinations of substitutions encompassed by the scope of the present invention are thus, for example, the following combinations of amino acid substitutions
(1) 82E, 86I, 89F, 120K; 26P and 153F;
(2) 82E, 86I, 89F, 120K, 51Q, 52A, 54S, 60M, and 63T;
(3) 82E, 86I, 89F, 120K, 2N, 10N, 37E, 92M, and 131M;
(4) 26P, 153F, 51Q, 52A, 54S, 60M, and 63T;
(5) 26P, 153F, 2N, 10N, 37E, 92M, and 131M;
(6) 51Q, 52A, 54S, 60M, 63T, 2N, 10N, 37E, 92M, and 131M;
(7) 82E, 86I, 89F, 120K, 51Q, 52A, 54S, 60M, 63T, 2N, 10N, 37E, 92M, and 131M;
(8) 26P, 153F, 51Q, 52A, 54S, 60M, 63T, 2N, 10N, 37E, 92M, and 131M;
(9) 82E, 86I, 89F, 120K, 26P, 153F, 51Q, 52A, 54S, 60M, 63T, 2N, 10N, 37E, 92M, and 131M;
(10) 86I, 89F, 161, 22G, 26Y, and 153F;
(11) 86I, 89F, 52A, 54S, 55A, 56F, and 63T;
(12) 86I, 89F, 11N, 14A, 34N, 42V, 51Q, 100T, 102E, 106E, 1081, 109A, 112N, 131M, 132G, 159K, 160G, 163R, and 165D;
(13) 161, 22G, 26Y, 153F, 52A, 54S, 55A, 56F, and 63T;
(14) 161, 22G, 26Y, 153F, 11N, 14A, 34N, 42V, 51Q, 100T, 102E, 106E, 1081, 109A, 112N, 131M, 132G, 159K, 160G, 163R, and 165D;
(15) 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(16) 86I, 89F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(17) 16I, 22G, 26Y, 153F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(18) 86I, 89F, 161, 22G, 26Y, 153F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(19) 15M, 52A, 54S, 59V, 63T, and 153S;
(20) 11H, 15M, 37R, 74V, 79R, 84L, 100M, 102E, 104W, 106G, 107G, 112N, 131T, 153S; 155R, 160R, and 163R
(21) 11H, 37R, 52A, 54S, 59V, 63T, 74V, 79R, 84L, 100M, 102E, 104W, 106G, 107G, 112N, 131T, 155R, 160R, and 163R; or
(22) 11H, 15M, 37R, 52A, 54S, 59V, 63T, 74V, 79R, 84L, 100M, 102E, 104W, 106G, 107G, 112N, 131T, 153S 155R, 160R, and 163R.

In various embodiments, the recombinant IFNa2 polypeptides differ from the amino acid sequences set forth in SEQ ID NO:2 (IFNa14) and 3 (IFNa16) and 14 (IFNa6) in at least one amino acid position. Preferably the sequence identity to the sequences set forth in SEQ ID Nos. 2, 3 and 14 is less than 99, preferably less than 97 %, more preferably less than 95%. In various embodiments, it may be preferred that the sequence identity to SEQ ID NO:1 is higher than to either one of SEQ ID Nos. 2, 3 and 14.

The fragments of the recombinant IFNa2 polypeptide referred to herein are functional fragments and are typically at least 100 amino acids in length, preferably at least 110, at least 120, at least 130, at least 140, at least 150, at least 160, 161, 162, 163 or 164 amino acids in length. Such fragments generally retain at least one of the substitutions such that they are not fragments of the wildtype sequence set forth in SEQ ID NO:1. The fragments of the invention are preferably designed such that they retain critical structural and functional determinants, such as the helix A (residues 8-21), AB loop (residues 30-48), helix B (residues 49-68), helix C (residues 78-97), helix D (residues 116-133) and helix E (residues 137-156) (positional numbering according to SEQ ID NO:1). Preferably also the amino acid stretch spanning amino acids 25-27, using positional numbering according to SEQ ID NO:1) is retained.

In various embodiments, the isolated polypeptide comprises less than 165 continuous amino acids of the amino acid sequence set forth in SEQ ID NO:1.

In some embodiments, the recombinant IFNa2 polypeptide fragment is derived from the amino acid sequence set forth in SEQ ID NO:1 including any one or more of the defined substitutions by any one or more of an N-terminal truncation, a C-terminal truncation or a deletion of one or more amino acids.

In various embodiments, the recombinant IFNa2 polypeptide has relative to a polypeptide having the amino acid sequence of SEQ ID NO:1 an affinity for IFNAR1 and/or IFNAR2 (interferon alpha receptor 1 and 2) or increased antiviral activity against HBV, HIV or any other pathogenic virus.

In another aspect, the invention also relates to a nucleic acid, nucleic acid molecule or isolated nucleic acid molecule encoding the isolated polypeptide as described herein. In one aspect, said nucleic acid is part of a vector. One aspect thus features a (nucleic acid) vector comprising a nucleic acid molecule according to the invention. The vector may be an expression vector and may comprise additional nucleic acid sequences necessary to facilitate its function in a host cell.

One further aspect of the invention relates to a host cell comprising a nucleic acid molecule according to the invention or a vector according to the invention. The host cell may be a prokaryotic host cell, for example an *E.coli* cell or may be an eukaryotic cells, such as a yeast cell or insect cell, including S. *cerevisiae* and Sf9 cells, or a mammalian cell.

In a still further aspect, the invention is directed to a method for the production of a polypeptide (isolated polypeptide) as described herein, comprising
(1) cultivating the host cell described herein under conditions that allow the expression of the polypeptide; and
(2) isolating the expressed polypeptide from the host cell.

The method may, in various embodiments, further comprise recovering the expressed peptide or protein from the host cell and/or the culture medium. In some embodiments of the methods described herein, the method further comprises secretion of the expressed recombinant peptide or protein into the culture medium by cultivating the host cell under conditions that allow secretion of the recombinant peptide or protein into the culture medium. To achieve this, the host cell may comprise further nucleic acid molecules that encode for components of a secretion system.

In various embodiments, the invention also relates to a pharmaceutical composition comprising the recombinant IFNa2 polypeptide of the invention, the nucleic acid of claim of the invention or the vector of the invention and, optionally, at least one pharmaceutically acceptable excipient.

The polypeptide or the pharmaceutical composition described herein may be for use in the treatment and/or prevention of a disease or disorder in a subject in need thereof, in particular the treatment and/or prevention of an infection by a pathogen. Said disease or disorder may include cancer like renal-cell carcinomas, cutaneous melanoma, hairy-cell leukemia, chronic myelogenous leukemia (CML), skin carcinomas, or hepatocellular carcinomas, immunological disorders, and infectious diseases, such as those caused by bacterial or viral pathogens or certain parasites. In various embodiments, the pathogen is a virus, for example, selected from the group consisting of influenza virus, corona virus, zika virus, dengue virus, west nile virus, ebola virus, hepatitis B virus, hepatitis D virus, hepatitis A virus, hepatitis E virus, adenovirus, herpes viruses (CMV, EBV, HSV, KSHV, VZV), RSV, BKV, JCV, and HIV.

In various embodiments, the subject is a mammal, preferably a human being.

It is understood that all combinations of the above disclosed embodiments are also intended to fall within the scope of the present invention.

### BRIEF DESCRIPTION OF THE DRAWINGS

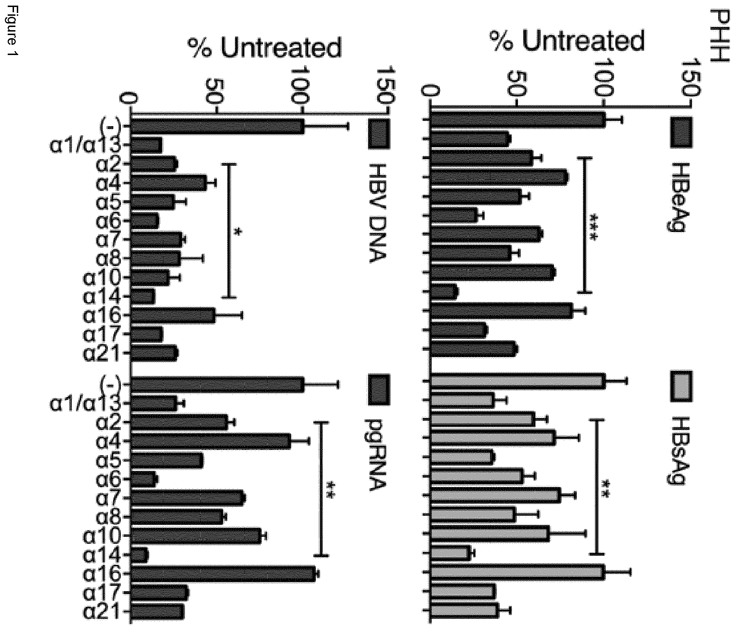

Figure 1: Effect of IFNα subtypes on HBV replication and gene expression *in vitro.* The levels of extracellular HBeAg, HBsAg, HBV core particle-associated-DNA and pregenomic RNA of HBV-infected primary human hepatocytes (PHH) treated with IFNα subtypes (0.5 ng/ml) were examined by ELISA and qPCR.

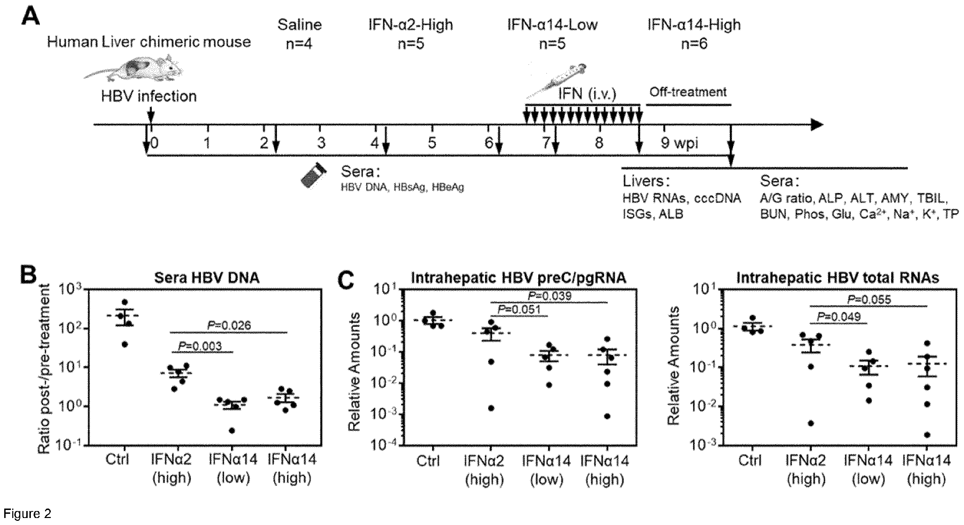

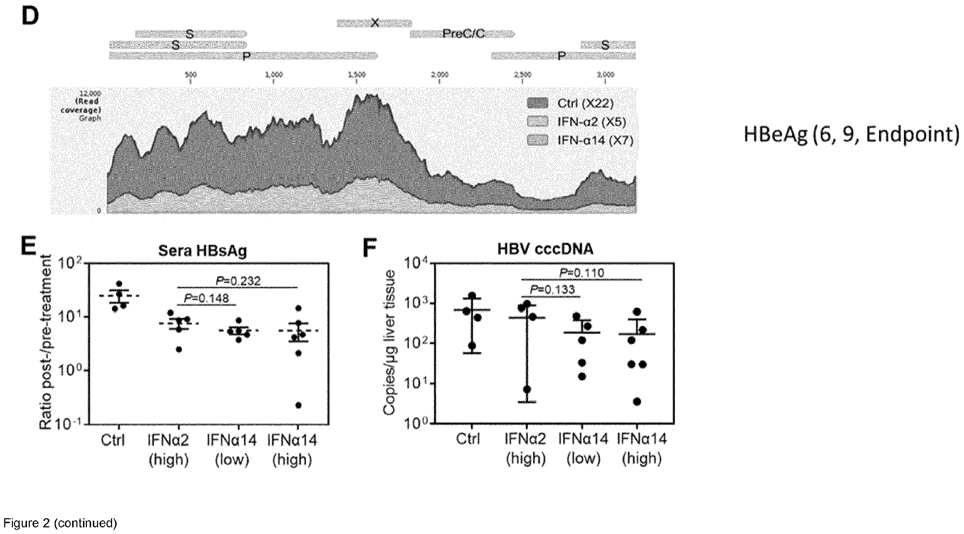

Figure 2: Potent suppression of established HBV infection and reduction of cccDNA amounts by IFNα14 *in vivo.* (A) Schematic model of study design. (B) The levels of sera HBV DNA before and after IFN/placebo treatment were examined. The data was shown as the ratio of post-/pre-treatment. (C) The levels of intrahepatic HBV preC/pgRNA and viral total RNAs at the endpoint were quantitated with specific primers. The data was shown as relative amounts. Sample X21 (Ctrl) was made relative to 1 for fold change analyses. Human albumin (hALB) was used as housekeeping gene. (D) HBV transcript-level quantification by nucleotide mapping in the indicated mice were analyzed by RNA sequencing. (E) The levels of sera HBsAg before and after IFN/placebo treatment were examined. The data was shown as the ratio of post-/pre-treatment. (F) The levels of intrahepatic HBV cccDNA at the endpoint were quantitated with specific primers. The data was shown as copies per µg liver tissue. Differences between groups are shown as P-values.

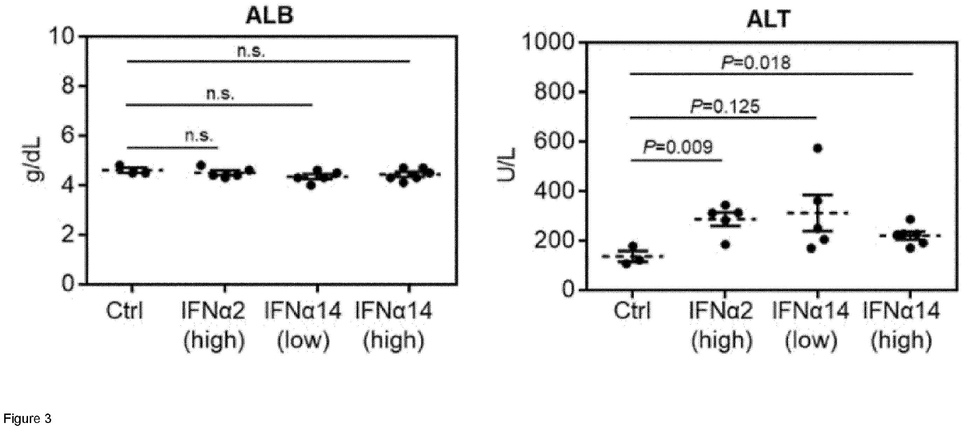

Figure 3: IFNα subtype treatment of HBV-infected mice with little side-effects *in vivo.* The serum levels of human albumin (ALB) and Alanine-Amino-Transferase (ALT) at the endpoint were quantitated. Differences between groups are shown as P-values.

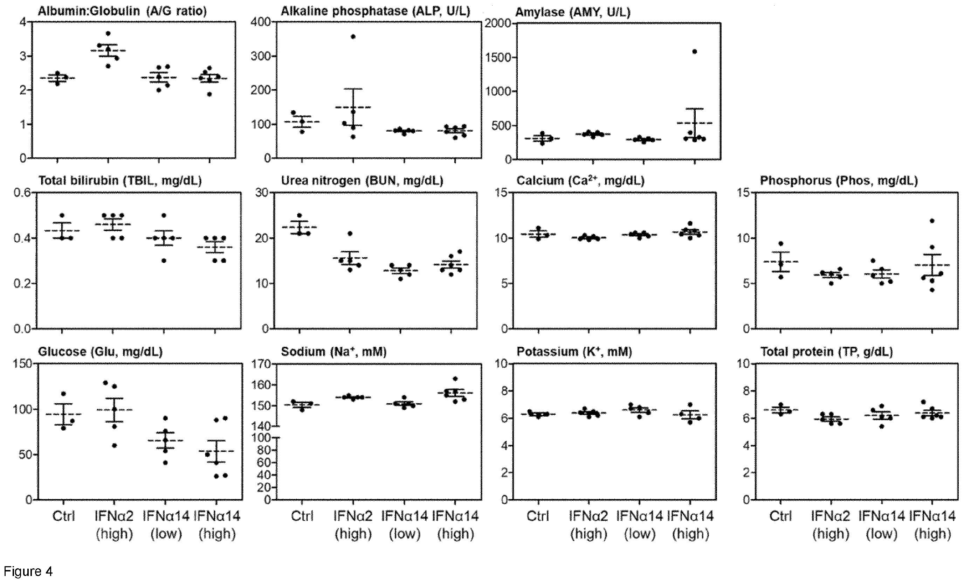

Figure 4: Blood chemistry analysis of the serum samples after IFNα subtype treatment of HBV-infected mice. The levels of indicated serum parameters at the endpoint were quantitated.

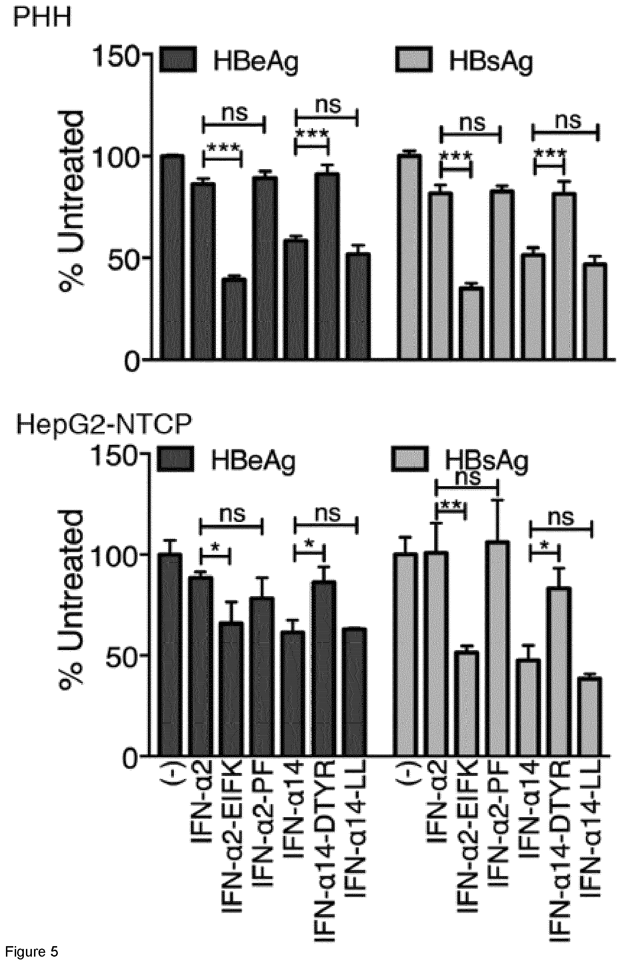

Figure 5: Effect of IFNa2 variants on HBV gene expression *in vitro.* The levels of extracellular HBeAg, and HBsAg of HBV-infected primary human hepatocytes (PHH) and HepG2-NTCP cells treated with IFNa2 variants (0.5 ng/ml) were examined by ELISA. IFNa2-EIFK corresponds to SEQ ID NO:4 and IFNa2-PF corresponds to SEQ ID NO:5.

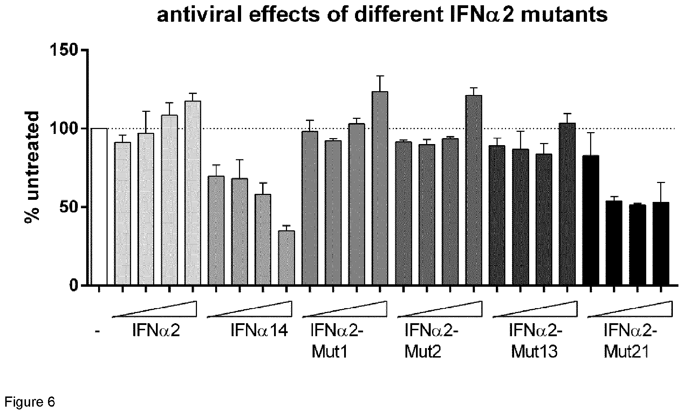

Figure 6: Effect of IFNa2 variants on HBV gene expression *in vitro.* The levels of extracellular HBsAg of HBV-infected primary human hepatocytes (PHH) treated with increasing concentrations of IFNa2 mutants were examined by ELISA and normalized to the untreated control (-, white bar). IFNa2-Mut2 corresponds to SEQ ID NO:4, IFNa2-Mut1 corresponds to SEQ ID NO:5, IFNa2-Mut13 corresponds to SEQ ID NO:6 and IFNa2-Mut21 corresponds to SEQ ID NO:8.

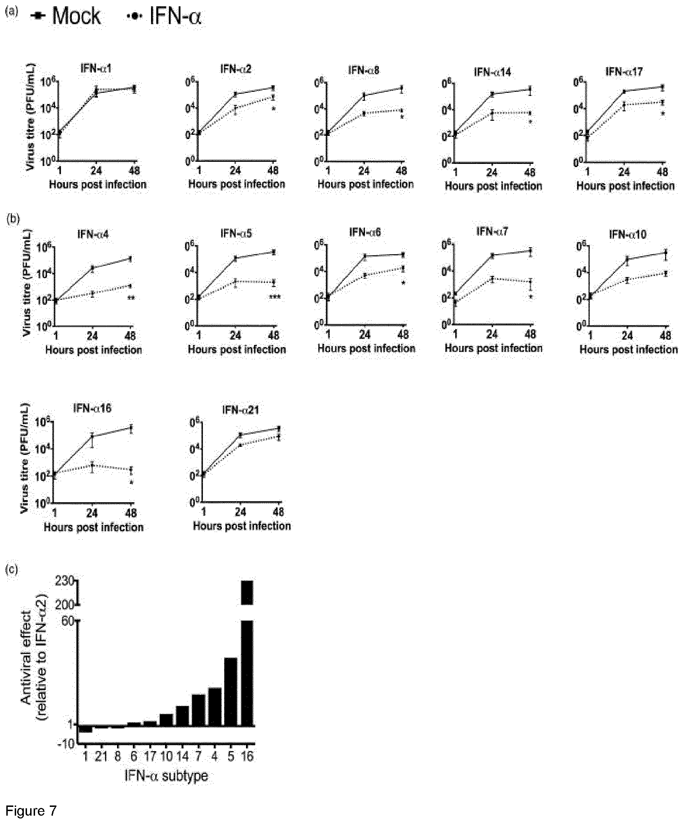

Figure 7: Inhibition of H3N2 IAV replication by IFNα subtypes. Tissues were treated with human IFNα subtypes (1000 U/mL) for 7 h before 1 × 10⁵ PFU/mL of H3N2 IAV were added to the medium. (A) Antiviral activity of IFNα subtypes, that are significantly induced during IAV infection. (B) Antiviral activity of the remaining IFNα subtypes, that are not induced during IAV infection. Viral titers were determined by plaque assay. Time points represent mean (± SEM). (C) Antiviral effects of IFNα subtypes against H3N2 were calculated over mock-treated tissue (n-fold reduction in viral titres) and compared to IFN-α2, at 48 hpi. *p < 0.05 **p < 0.01 ***p < 0.001; two-way ANOVA multiple comparison test.

### DETAILED DESCRIPTION OF THE INVENTION

The terms used herein have, unless explicitly stated otherwise, the meanings as commonly understood in the art.

"At least one", as used herein, relates to one or more, in particular 1, 2, 3, 4, 5, 6, 7, 8, 9, 10 or more.

"Isolated" as used herein in relation to a molecule means that said molecule has been at least partially separated from other molecules it naturally associates with or other cellular components. "Isolated" may mean that the molecule has been purified to separate it from other molecules and components, such as other proteins and nucleic acids and cellular debris.

"Recombinant", as used herein, relates to proteins that result from the expression of recombinant DNA within living cells are termed. Recombinant DNA is in turn DNA formed by genetic engineering that is artificially created and does not occur in nature. In the present invention, the recombinant proteins are preferably chimeric or hybrid in nature in that they comprise sequence elements of two different proteins in a combination that does not naturally occur.

"Nucleic acid" as used herein includes all natural forms of nucleic acids, such as DNA and RNA. Preferably, the nucleic acid molecules of the invention are DNA.

The term "peptide" is used throughout the specification to designate a polymer of amino acid residues connected to each other by peptide bonds. A peptide according to the present invention may have 2-100 amino acid residues. The terms "protein" and "polypeptide" are used interchangeably throughout the specification to designate a polymer of amino acid residues connected to each other by peptide bonds. A protein or polypeptide according to the present invention has preferably 100 or more amino acid residues.

The term "an N-terminal fragment" relates to a peptide or protein sequence which is in comparison to a reference peptide or protein sequence C-terminally truncated, such that a contiguous amino acid polymer starting from the N-terminus of the peptide or protein remains. Similarly, the term "a C-terminal fragment" relates to a peptide or protein sequence which is in comparison to a reference peptide or protein sequence N-terminally truncated, such that a contiguous amino acid polymer starting from the C-terminus of the peptide or protein remains.

"IFNa2" or "interferon alpha 2", as used herein, relates to mature human interferon alpha 2 having the amino acid sequence set forth in SEQ ID NO:1 or a naturally occurring variant thereof. Such "variants" are homologues of human IFNa2 that have typically at least one but not more than 3 amino acid exchanges that occur naturally and do not significantly impair functionality of the protein. The sequence identity is thus typically higher than 98%, often more than 95%. Such variants do not cover the recombinant polypeptides of the invention. It is understood that the polypeptides described herein may comprise additional amino acid sequences on the N- or C-terminus, such as a signal peptide, which is also present in naturally occurring IFNa2 prior to post-translational processing. Other elements that may be present include various tags or markers that facilitate expression, purification and/or detection, as well as protease recognition sites that allow cleavage of such additional sequence elements.

Generally, the skilled person understands that for putting the present invention into practice any nucleotide sequence described herein may comprise an additional start and/or stop codon or that a start and/or stop codon included in any of the sequences described herein may be deleted, depending on the nucleic acid construct used. The skilled person will base this decision, e.g., on whether a nucleic acid sequence comprised in the nucleic acid molecule of the present invention is to be translated and/or is to be translated as a fusion protein. In various embodiments, the polypeptides of the invention additionally comprise the amino acid M on the N-terminus. In various other embodiments, a signal peptide may be present on the N-terminus.

The present invention is based on the inventor's surprising finding that chimeric variants of IFNa2 (SEQ ID NO:1) that comprise substitutions at certain positions that are derived from either IFNa6, IFNa14 or IFNa16 provide for increased anti-viral activity relative to IFNa2 wt.

Thus, in a first aspect, the present invention relates to a recombinant interferon alpha 2 (IFNa2) polypeptide comprising an amino acid sequence having at least 80 %, at least 85 %, at least 90 %, at least 95%, at least 97.5%, at least 99% or at least 99.5% sequence identity over its entire length with the amino acid sequence set forth in SEQ ID NO:1 and comprising one or more amino acid substitution(s) in any one or more of the positions corresponding to positions 2, 10, 11, 14, 15, 16, 22, 26, 34, 37, 42, 51, 52, 54, 55, 56, 59, 60, 63, 74, 79, 82, 84, 86, 89, 92, 100, 102, 104, 106, 107, 108, 109, 112, 120, 131, 132, 153, 155 159, 160, 163, and 165 of SEQ ID NO:1, or fragments or variants thereof. The polypeptide may be an isolated polypeptide, as described herein.

Any positional numbering used herein refers, if not explicitly indicated otherwise, to the positional numbering of SEQ ID NO:1. As described above, the corresponding positions in a given amino acid sequence may be identified using alignments.

The polypeptide does not include the amino acid sequence as set forth in SEQ ID NO:1, but comprises a variant thereof that comprises at least one substitution as defined above. Similarly, the polypeptide does not include the amino acid sequence as set forth in SEQ ID NO:2, SEQ ID NO:3 or SEQ ID NO:14, but comprises a variant thereof that comprises at least one substitution, preferably in positions other than those defined above.

The amino acid sequence of the recombinant polypeptide has, over its entire length, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95%, at least 95.6 %, at least 96.2 %, at least 96.8 %, at least 97.2 %, at least 97.6%, at least 98.2 %, at least 98.8 %, or 99.4% sequence identity with the corresponding part of the amino acid sequence set forth in SEQ ID NO:1. In various embodiments, the amino acid sequence of the recombinant polypeptide is of the same length as the sequence set forth in SEQ ID NO:1. In other embodiments, it is a shortened fragment thereof that may be obtainable by deletions/truncations. In various embodiments, the sequence of the amino acid sequence with the exception of the above-indicated positions that may be substituted, i.e. the remainder of the amino acid sequence that is not substituted or deleted, is essentially identical to the sequence set forth in SEQ ID NO:1, i.e. has sequence identities of at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95%, at least 96 %, at least 97 %, at least 97.5%, at least 98.2 %, at least 98.8 %, at least 99%, at least 99.4% or 100% with the sequence of SEQ ID NO:1.

Determination of the sequence identity of nucleic acid or amino acid sequences can be done by a sequence alignment based on well-established and commonly used BLAST algorithms (See, e.g. Altschul, S.F., Gish, W., Miller, W., Myers, E.W. & Lipman, D.J. (1990) "Basic local alignment search tool." J. Mol. Biol. 215:403-410, and Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402). Such an alignment is based on aligning similar nucleotide or amino acid sequences stretches with each other. Another algorithm known in the art fo said purpose is the FASTA-Algorithmus. Alignments, in particular multiple sequence comparisons, are typically done by using computer programs. Commonly used are the Clustal series (See, e.g., Chenna et al. (2003): Multiple sequence alignment with the Clustal series of programs. Nucleic Acid Research 31, 3497-3500), T-Coffee (See, e.g., Notredame et al. (2000): T-Coffee: A novel method for multiple sequence alignments. J. Mol. Biol. 302, 205-217) or programs based on these known programs or algorithms.

Also possible are sequence alignments using the computer program Vector NTI® Suite 10.3 (Invitrogen Corporation, 1600 Faraday Avenue, Carlsbad, CA, USA) with the set standard parameters, with the AlignX module for sequence comparisons being based on the ClustalW. If not indicated otherwise, the sequence identity is determined using the BLAST algorithm.

Such a comparison also allows determination of the similarity of the compared sequences. Said similarity is typically expressed in percent identify, i.e. the portion of identical nucleotides/amino acids at the same or corresponding (in an alignment) sequence positions relative to the total number of the aligned nucleotides/amino acids. For example, if in an alignment 90 amino acids of a 100 aa long query sequence are identical to the amino acids in corresponding positions of a template sequence, the sequence identity is 90%. The broader term "homology" additionally considers conserved amino acid substitutions, i.e. amino acids that are similar in regard to their chemical properties, since those typically have similar chemical properties in a protein. Accordingly, such homology can be expressed in percent homology. If not indicated otherwise, sequence identity and sequence homology relate to the entire length of the aligned sequence.

In the context of the present invention, the feature that an amino acid position corresponds to a numerically defined position in SEQ ID NO:1 means that the respective position correlates to the numerically defined position in SEQ ID NO:1 in an alignment obtained as described above.

In various embodiments, the amino acid sequence comprised in the polypeptide of the invention is a variant of human IFNa2 with the amino acid set forth in SEQ ID NO:1 and comprises, relative to the amino acid sequence of SEQ ID NO:1, one or more amino acid substitution(s) or deletion(s) in any one of the positions corresponding to positions 2, 10, 11, 14, 15, 16, 22, 26, 34, 37, 42, 51, 52, 54, 55, 56, 59, 60, 62, 63, 74, 79, 82, 84, 86, 89, 92, 100, 102, 104, 106, 107, 108, 109, 112, 120, 131, 132, 153, 155, 159, 160, 163, and 165 of SEQ ID NO:1, for example in any one or more of the positions corresponding to positions 2, 10, 11, 14, 15, 16, 22, 26, 34, 37, 42, 51, 52, 54, 55, 56, 59, 60, 63, 74, 79, 82, 86, 89, 92, 100, 102, 104, 106, 107, 108, 109, 112, 120, 131, 132, and 153, preferably 15, 16, 22, 26, 51, 52, 54, 55, 56, 59, 60, 63, 82, 86, 89, 120, and 153, or, preferably, 15, 16, 22, 26, 82, 86, 89, 120, and 153.

The recombinant IFNa2 polypeptide may comprise amino acid substitution(s) in
(i) any one or more of the positions corresponding to positions 2, 10, 26, 37, 51, 52, 54, 60, 63, 82, 86, 89, 92, 120, 131, and 153 of SEQ ID NO:1;
(ii) any one or more of the positions corresponding to positions 11, 14, 16, 22, 26, 34, 42, 51, 52, 54, 55, 56, 63, 86, 89, 100, 102, 106, 108, 109, 112, 131, 132, 153, 159, 160, 163, and 165 of SEQ ID NO:1;
(iii) any one or more of the positions corresponding to positions 11, 15, 37, 52, 54, 59, 63, 74, 79, 84, 100, 102, 104, 106, 107, 112, 131, 153, 155, 160, and 163 of SEQ ID NO:1;
(iv) the positions corresponding to positions 82, 86, 89, and 120 of SEQ ID NO:1;
(v) the positions corresponding to 26 and 153 of SEQ ID NO:1;
(vi) the positions corresponding to positions 26, 82, 86, 89, 120, and 153 of SEQ ID NO:1;
(vii) the positions corresponding to positions 51, 52, 54, 60, and 63 of SEQ ID NO:1;
(viii) the positions corresponding to positions 2, 10, 37, 92, and 131 of SEQ ID NO:1;
(ix) the positions corresponding to positions 86 and 89 of SEQ ID NO:1;
(x) the positions corresponding to positions 16, 22, 26, and 153 of SEQ ID NO:1;
(xi) the positions corresponding to positions 16, 22, 26, 86, 89, and 153 of SEQ ID NO:1;
(xii) the positions corresponding to positions 52, 54, 55, 56, and 63 of SEQ ID NO:1;
(xiii) the positions corresponding to positions 11, 14, 34, 42, 51, 100, 102, 106, 108, 109, 112, 131, 132, 159, 160, 163, and 165 of SEQ ID NO:1;
(xiv) the positions corresponding to positions 15 and 153 of SEQ ID NO:1;
(xv) the positions corresponding to positions 52, 54, 59, and 63 of SEQ ID NO:1; or
(xvi) the positions corresponding to positions 11, 37, 74, 79, 84, 100, 102, 104, 106, 107, 112, 131, 155, 160 and 163 of SEQ ID NO:1.

Also possible are combinations of the above-indicated sets (iv)-(viii) and of sets (ix)-(xiii) and of sets (xiv)-(xvi) .

In the amino acid sequence as set forth in SEQ ID NO:1, the above-identified positions are occupied by the following amino acid residues: D2, G10, S11, T14, L15, M16, R22, L26, H34, G37, E42, E51, T52, P54, V55, L56, M59, I60, I63, A74, T79, D82, F84, T86, Y89, L92, 1100, G102, G104, T106, E107, T108, P109, K112, R120, K131, E132, L153, T155, E159, S160, S163, and E165. It is to be noted that IFNa2 comprises, relative to IFNa6, IFNa14 and IFNa16, a deletion in the position that corresponds to position 44 in IFNa6, IFNa14 and IFNa16. This means that, for example, position 51 in IFNa2 corresponds to position 52 in IFNa6, IFNa14 and IFNa16.

"Amino acid substitution", as used herein, relates to modification of the sequence such that the amino acid residue occurring at the corresponding position in SEQ ID NO:1 is replaced by another amino acid residue. The amino acid residue for substitution is typically selected from the 20 proteinogenic amino acids G, A, V, L, I, F, C, M, P, R, K, H, N, Q, D, E, S, T, W, and Y. Accordingly, if a position in SEQ ID NO:1 is occupied by any one of these 20 amino acid residues, its substitution means that it is replaced by any one of the other 19 amino acids listed above.

While in principle all of the positions defined herein may be mutated such that the naturally occurring amino acid residue is replaced by any other amino acid residue, it can be preferable, in various embodiments, that the substitutions are such that the position is mutated to the corresponding amino acid residue occurring in human IFNa6 (SEQ ID NO:14), IFNa14 (SEQ ID NO:2) or human IFNa16 (SEQ ID NO:3)

In any of the embodiments described herein, the amino acid substitution(s) in the above-listed positions may, without limitation, be selected from the group consisting of: 2N, 10N, 11N, 11H, 14A, 15M, 161, 22G, 26P, 26Y, 34N, 37E, 37R, 42V, 51Q, 52A, 54S, 55A, 56F, 59V, 60M, 63T, 74V, 79R, 82E, 84L, 86I, 89F, 92M, 100T, 100M, 102E, 104W, 106G, 106E, 107G, 1081, 109A, 112N, 120K, 131M, 131T, 132G, 153F, 153S, 155R, 159K, 160G, 160R, 163R, and 165D using the positional numbering of SEQ ID NO:1. These substitutions are the amino acids found in IFNa6, IFNa14 or IFNa16 at the corresponding positions.

Accordingly, in various embodiments, the recombinant IFNa2 polypeptide comprises, using the numbering of SEQ ID NO:1, any one or more amino acid substitution(s) that exchange the amino acid of IFNa2 to the amino acid of IFNa14 at the corresponding positions, namely substitutions selected from the group consisting of 2N, 10N, 26P, 37E, 51Q, 52A, 54S, 60M, 63T, 82E, 86I, 89F, 92M, 120K, 131M, and 153F.

In various other embodiments, the recombinant IFNa2 polypeptide comprises, using the numbering of SEQ ID NO:1, any one or more amino acid substitution(s) that exchange the amino acid of IFNa2 to the amino acid of IFNa16 at the corresponding positions, namely substitutions selected from the group consisting of 11N, 14A, 161, 22G, 26Y, 34N, 42V, 51Q, 52A, 54S, 55A, 56F, 63T, 86I, 89F, 100T, 102E, 106E, 1081, 109A, 112N, 131M, 132G, 153F, 159K, 160G, 163R, and 165D.

In various other embodiments, the recombinant IFNa2 polypeptide comprises, using the numbering of SEQ ID NO:1, any one or more amino acid substitution(s) that exchange the amino acid of IFNa2 to the amino acid of IFNa6 at the corresponding positions, namely substitutions selected from the group consisting of 11H, 15M, 37R, 52A, 54S, 59V, 63T, 74V, 79R, 84L, 100M, 102E, 104W, 106G, 107G, 112N, 131T, 153S, 155R, 160R, and 163R.

In various embodiments, the polypeptide comprises any two or more, three or more, four or more, five or more, six or more, seven or more, eight or more, nine or more or ten or more of the amino acid substitution(s) identified herein.

In various embodiments, the recombinant IFNa2 polypeptide comprises, using the numbering of SEQ ID NO:1, any one or more amino acid substitution(s) that exchange the amino acid of IFNa2 to the amino acid of IFNa14 at the corresponding positions in IFNAR1 binding/contacting sites, namely the amino acid substitutions 82E, 86I, 89F, and 120K.

In various embodiments, the recombinant IFNa2 polypeptide comprises, using the numbering of SEQ ID NO:1, any one or more amino acid substitution(s) that exchange the amino acid of IFNa2 to the amino acid of IFNa14 at the corresponding positions in IFNAR2 binding/contacting sites, namely the amino acid substitutions 26P and 153F.

In various embodiments, the recombinant IFNa2 polypeptide comprises, using the numbering of SEQ ID NO:1, any one or more amino acid substitution(s) that exchange the amino acid of IFNa2 to the amino acid of IFNa14 at the corresponding positions in IFNAR1 and IFNAR2 binding/contacting sites, namely the amino acid substitutions 26P, 82E, 86I, 89F, 120K, and 153F.

In various embodiments, the recombinant IFNa2 polypeptide comprises, using the numbering of SEQ ID NO:1, any one or more amino acid substitution(s) that exchange the amino acid of IFNa2 to the amino acid of IFNa14 at the corresponding positions in the tunable loop region, namely the amino acid substitutions 51Q, 52A, 54S, 60M, and 63T.

In various embodiments, the recombinant IFNa2 polypeptide comprises, using the numbering of SEQ ID NO:1, any one or more amino acid substitution(s) that exchange the amino acid of IFNa2 to the amino acid of IFNa14 at the corresponding positions, namely the amino acid substitutions 2N, 10N, 37E, 92M, and 131M. These mutations may be individually or in any combination be combined with the mutations in the IFNAR1 binding site, IFNAR2 binding site and/or tunable loop region described above.

In various embodiments, the recombinant IFNa2 polypeptide comprises, using the numbering of SEQ ID NO:1, any one or more amino acid substitution(s) that exchange the amino acid of IFNa2 to the amino acid of IFNa16 at the corresponding positions in IFNAR1 binding/contacting sites, namely the amino acid substitutions 86I and 89F.

In various embodiments, the recombinant IFNa2 polypeptide comprises, using the numbering of SEQ ID NO:1, any one or more amino acid substitution(s) that exchange the amino acid of IFNa2 to the amino acid of IFNa16 at the corresponding positions in IFNAR2 binding/contacting sites, namely the amino acid substitutions 161, 22G, 26Y, and 153F.

In various embodiments, the recombinant IFNa2 polypeptide comprises, using the numbering of SEQ ID NO:1, any one or more amino acid substitution(s) that exchange the amino acid of IFNa2 to the amino acid of IFNa16 at the corresponding positions in IFNAR1 and IFNAR2 binding/contacting sites, namely the amino acid substitutions 161, 22G, 26Y, 861, 89F, and 153F.

In various embodiments, the recombinant IFNa2 polypeptide comprises, using the numbering of SEQ ID NO:1, any one or more amino acid substitution(s) that exchange the amino acid of IFNa2 to the amino acid of IFNa16 at the corresponding positions in the tunable loop region, namely the amino acid substitutions 52A, 54S, 55A, 56F, and 63T.

In various embodiments, the recombinant IFNa2 polypeptide comprises, using the numbering of SEQ ID NO:1, any one or more amino acid substitution(s) that exchange the amino acid of IFNa2 to the amino acid of IFNa16 at the corresponding positions, namely the amino acid substitutions 11N, 14A, 34N, 42V, 51Q, 100T, 102E, 106E, 1081, 109A, 112N, 131M, 132G, 159K, 160G, 163R, and 165D. These mutations may be individually or in any combination be combined with the mutations in the IFNAR1 binding site, IFNAR2 binding site and/or tunable loop region described above.

In various embodiments, the recombinant IFNa2 polypeptide comprises, using the numbering of SEQ ID NO:1, any one or more amino acid substitution(s) that exchange the amino acid of IFNa2 to the amino acid of IFNa6 at the corresponding positions in IFNAR2 binding/contacting sites, namely the amino acid substitutions 15M and 153S.

In various embodiments, the recombinant IFNa2 polypeptide comprises, using the numbering of SEQ ID NO:1, any one or more amino acid substitution(s) that exchange the amino acid of IFNa2 to the amino acid of IFNa6 at the corresponding positions in the tunable loop region, namely the amino acid substitutions 52A, 54S, 59V, and 63T.

In various embodiments, the recombinant IFNa2 polypeptide comprises, using the numbering of SEQ ID NO:1, any one or more amino acid substitution(s) that exchange the amino acid of IFNa2 to the amino acid of IFNa6 at the corresponding positions, namely the amino acid substitutions 11H, 37R, 74V, 79R, 84L, 100M, 102E, 104W, 106G, 107G, 112N, 131T, 155R, 160R, and 163R. These mutations may be individually or in any combination be combined with the mutations in the IFNAR2 binding site and/or tunable loop region described above.

In such instances where the above sets are combined, the recombinant IFNa2 polypeptide may comprise, using the numbering of SEQ ID NO:1,
(A) any combination of two or more of the following sets of amino acid substitution(s)
   (1) 82E, 86I, 89F, and 120K;
   (2) 26P and 153F;
   (3) 51Q, 52A, 54S, 60M, and 63T;
   (4) 2N, 10N, 37E, 92M, and 131M;
(B) any combination of two or more of the following sets of amino acid substitution(s)
   (1) 861 and 89F;
   (2) 161, 22G, 26Y, and 153F;
   (3) 52A, 54S, 55A, 56F, and 63T;
   (4) 11N, 14A, 34N, 42V, 51Q, 100T, 102E, 106E, 1081, 109A, 112N, 131M, 132G, 159K, 160G, 163R, and 165D;.
(C) any combination of two or more of the following sets of amino acid substitution(s)
   (1) 15M and 153S;
   (2) 52A, 54S, 59V, and 63T;
   (3) 11H, 37R, 74V, 79R, 84L, 100M, 102E, 104W, 106G, 107G, 112N, 131T, 155R, 160R, and 163R.

Concrete combinations of substitution sets encompassed by the scope of the present invention are thus, without limitation, the following combinations of amino acid substitutions
(1) 82E, 86I, 89F, 120K; 26P and 153F;
(2) 82E, 86I, 89F, 120K, 51Q, 52A, 54S, 60M, and 63T;
(3) 82E, 86I, 89F, 120K, 2N, 10N, 37E, 92M, and 131M;
(4) 26P, 153F, 51Q, 52A, 54S, 60M, and 63T;
(5) 26P, 153F, 2N, 10N, 37E, 92M, and 131M;
(6) 51Q, 52A, 54S, 60M, 63T, 2N, 10N, 37E, 92M, and 131M;
(7) 82E, 86I, 89F, 120K, 51Q, 52A, 54S, 60M, 63T, 2N, 10N, 37E, 92M, and 131M;
(8) 26P, 153F, 51Q, 52A, 54S, 60M, 63T, 2N, 10N, 37E, 92M, and 131M;
(9) 82E, 86I, 89F, 120K, 26P, 153F, 51Q, 52A, 54S, 60M, 63T, 2N, 10N, 37E, 92M, and 131M;
(10) 86I, 89F, 161, 22G, 26Y, and 153F;
(11) 86I, 89F, 52A, 54S, 55A, 56F, and 63T;
(12) 86I, 89F, 11N, 14A, 34N, 42V, 51Q, 100T, 102E, 106E, 1081, 109A, 112N, 131M, 132G, 159K, 160G, 163R, and 165D;
(13) 161, 22G, 26Y, 153F, 52A, 54S, 55A, 56F, and 63T;
(14) 16I, 22G, 26Y, 153F, 11N, 14A, 34N, 42V, 51Q, 100T, 102E, 106E, 1081, 109A, 112N, 131M, 132G, 159K, 160G, 163R, and 165D;
(15) 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(16) 86I, 89F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(17) 16I, 22G, 26Y, 153F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(18) 86I, 89F, 161, 22G, 26Y, 153F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(19) 15M, 52A, 54S, 59V, 63T, and 153S;
(20) 11H, 15M, 37R, 74V, 79R, 84L, 100M, 102E, 104W, 106G, 107G, 112N, 131T, 153S, 155R, 160R, and 163R;
(21) 11H, 37R, 52A, 54S, 59V, 63T, 74V, 79R, 84L, 100M, 102E, 104W, 106G, 107G, 112N, 131T, 155R, 160R, and 163R; or
(22) 11H, 15M, 37R, 52A, 54S, 59V, 63T, 74V, 79R, 84L, 100M, 102E, 104W, 106G, 107G, 112N, 131T, 153S, 155R, 160R, and 163R;

As the amino acid sequence of IFNa2 is used as a scaffold for all substitutions, the recombinant IFNa2 polypeptides typically differ from the amino acid sequences set forth in SEQ ID NO:2 (IFNa14), SEQ ID NO:3 (IFNa16) and SEQ ID NO:14 (IFNa6) in at least one amino acid position, typically in at least 10 amino acid positions. These positions in which they differ may be outside of the positions defined above, but may also include certain of the above positions where the amino acid of IFNa2 is retained. Preferably the sequence identity to the sequences set forth in SEQ ID Nos. 2 and 3 is less than 99, preferably less than 97 %, more preferably less than 95% and may be as low as 83 %, for example about 84 or about 85 %. "About", as used herein, in relation to a numerical value of sequence identity relates to said value ±1%. In various embodiments, the sequence identity of the polypeptide of the invention to SEQ ID NO:1 is higher than to either one of SEQ ID Nos. 2, 3, and 14.

The fragments of the recombinant IFNa2 polypeptide referred to herein are functional fragments and are typically at least 100 amino acids in length, preferably at least 110, at least 120, at least 130, at least 140, at least 150, at least 160, 161, 162, 163 or 164 amino acids in length. Such fragments generally retain at least one of the substitutions such that they are not fragments of the wildtype sequence set forth in SEQ ID NO:1. The fragments of the invention are preferably designed such that they retain critical structural and functional determinants, such as the helix A (residues 8-21), AB loop (residues 30-48), helix B (residues 49-68), helix C (residues 78-97), helix D (residues 116-133) and helix E (residues 137-156) (positional numbering according to SEQ ID NO:1).Preferably also the amino acid stretch spanning amino acids 25-27, using positional numbering according to SEQ ID NO:1) is retained.

In various embodiments, the isolated polypeptide comprises less than 165 continuous amino acids of the amino acid sequence set forth in SEQ ID NO:1.

In some embodiments, the recombinant IFNa2 polypeptide fragment is derived from the amino acid sequence set forth in SEQ ID NO:1 including any one or more of the defined substitutions by any one or more of an N-terminal truncation, a C-terminal truncation or a deletion of one or more amino acids.

In various embodiments, the polypeptide comprises the sequence set forth in any one of SEQ ID Nos. 4-13. In further embodiments, also encompassed are variants of these sequences that have at least 80 %, at least 85 %, at least 90 %, at least 95%, at least 97.5%, at least 99% or at least 99.5% sequence identity with the amino acid sequence set forth in the respective template sequence of any one of SEQ ID Nos. 4-13. These variants include truncated versions of the sequences set forth in SEQ ID Nos. 4-13, for example N- or C-terminal truncations, with these truncations typically being 1-10, preferably 1-5 amino acids in length.

In various embodiments, also contemplated herein are naturally occurring variants of the IFNa2 wildtype sequence set forth in SEQ ID NO:1. Such naturally occurring variants may comprise several amino acid substitutions that typically do not impair functionality. Such variants thus have typically more than 80 % sequence identity to SEQ ID NO:1 and may additionally comprise any one or more of the substitutions described herein. Such known variations include, but are not limited to R23K and H34R, as well as Q62L and K70M. These variations may be included in addition to the substitutions defined herein.

The polypeptide may, in various embodiments, comprises additional amino acids except the sequences specified herein. In various embodiments, the polypeptide comprises as the first, N-terminal amino acid the residue M. If this is not present within the specific sequences disclosed herein, it may be artificially added, if desired, in particular to facilitate expression in a host cell.

The polypeptides of the invention may comprise additional sequences that may include, among others, an affinity tag and/or a protease recognition and cleavage site as well as other proteins/polypeptides to which the polypeptides of the invention are fused, thus forming a fusion protein. Examples of such proteins to which the polypeptides of the invention may be fused include, without limitation, albumins and antibodies, as well as antibody fragments or antibody-like molecules and antibody derivatives.

The term "affinity tag" as used herein relates to entities which are coupled to a molecule of interest and allow enrichment of the complex between the molecule of interest and the affinity tag using an affinity tag receptor. In certain embodiments affinity tags may be selected from the group consisting of the Strep-tag® or Strep-tag® II, the myc-tag, the FLAG-tag, the His-tag, the small ubiquitin-like modifier (SUMO) tag, the covalent yet dissociable NorpD peptide (CYD) tag, the heavy chain of protein C (HPC) tag, the calmodulin binding peptide (CBP) tag, or the HA-tag or proteins such as Streptavidin binding protein (SBP), maltose binding protein (MBP), and glutathione-S-transferase.

The term "protease (recognition and) cleavage site" refers to a peptide sequence which can be cleaved by a selected protease thus allowing the separation of peptide or protein sequences which are interconnected by a protease cleavage site. In certain embodiments the protease cleavage site is selected from the group consisting of a Factor Xa, a tobacco edge virus (TEV) protease, a enterokinase, a SUMO Express protease, an Arg-C proteinase, an Asp-N endopeptidases, an Asp-N endopeptidase + N-terminal Glu, a caspase 1, a caspase 2, a caspase 3, a caspase 4, a caspase 5, a caspase 6, a caspase 7, a caspase 8, a caspase 9, a caspase 10, a chymotrypsin-high specificity, a chymotrypsin-low specificity, a clostripain (Clostridiopeptidase B), a glutamyl endopeptidase, a granzyme B, a pepsin, a proline-endopeptidase, a proteinase K, Welqut protease, Clean Cut protease, a staphylococcal peptidase I, a Thrombin, a Trypsin, intein, and a Thermolysin cleavage site. It can be preferred, in some embodiments, to design the protease recognition site such that as few amino acids as possible of the recognition and cleavage site remain attached to the peptide or protein of interest.

In various embodiments, the polypeptide may be derivatized or conjugated to another chemical moiety, said derivatization/conjugation including, amongst others, PEGylation, glycosylation. In particular, PEGylation, i.e. covalent coupling to polyethylene glycol of various molecular weights, is known as a means to alter pharmaokinetics of such compounds.

The invention further relates to the nucleic acid, in particular the isolated nucleic acid molecule, encoding the polypeptide as described above. If the polypeptide comprises in addition to the amino acid sequence specified herein optional further amino acid sequences, all of these amino acid sequences are typically linked by peptide bonds and expressed as a single fusion protein. To facilitate said expression, the nucleic acid molecule comprises a nucleotide sequences encoding all amino acid sequences, with said nucleotide sequences being operably linked to allow expression of the single fusion protein comprising all afore-mentioned amino acid sequences.

The term "operably linked" in the context of nucleic acid sequences means that a first nucleic acid sequence is linked to a second nucleic acid sequence such that the first nucleic acid sequence is placed in a functional relationship with the second nucleic acid sequence. For instance, a promoter sequence is operably linked to a coding sequence of a heterologous gene if the promoter can initiate the transcription of the coding sequence. In a further context, a sequence encoding the claimed IFNa variants is linked such to another amino acid sequence, that if the two sequences are translated a single peptide/protein chain is obtained.

In certain embodiments, the above defined nucleic acid molecules may be comprised in a vector, for example a cloning or expression vector. Generally, the nucleic acid molecules of the invention can also be part of a vector or any other kind of cloning vehicle, including, but not limited to a plasmid, a phagemid, a phage, a baculovirus, a cosmid, or an artificial chromosome. Generally, a nucleic acid molecule disclosed in this application may be "operably linked" to a regulatory sequence (or regulatory sequences) to allow expression of this nucleic acid molecule.

Such cloning vehicles can include, besides the regulatory sequences described above and a nucleic acid sequence of the present invention, replication and control sequences derived from a species compatible with the host cell that is used for expression as well as selection markers conferring a selectable phenotype on transformed or transfected cells. Large numbers of suitable cloning vectors are known in the art, and are commercially available.

In certain embodiments the nucleic acid molecules disclosed herein are comprised in a cloning vector. In some embodiments the nucleic acid molecules disclosed herein are comprised in an expression vector. The vectors may comprise regulatory elements for replication and selection markers. In certain embodiments, the selection marker may be selected from the group consisting of genes conferring ampicillin, kanamycin, chloramphenicol, tetracycline, blasticidin, spectinomycin, gentamicin, hygromycin, and zeocin resistance. In various other embodiments, the selection may be carried out using antibiotic-free systems, for example by using toxin/antitoxin systems, cer sequence, triclosan, auxotrophies or the like. Suitable methods are known to those skilled in the art.

The above-described nucleic acid molecule of the present invention, comprising a nucleic acid sequence encoding for the polypeptide of the invention, if integrated in a vector, must be integrated such that the polypeptide can be expressed. Therefore, a vector of the present invention comprises sequence elements which contain information regarding to transcriptional and/or translational regulation, and such sequences are "operably linked" to the nucleotide sequence encoding the polypeptide. An operable linkage in this context is a linkage in which the regulatory sequence elements and the sequence to be expressed are connected in a way that enables gene expression. The precise nature of the regulatory regions necessary for gene expression may vary among species, but in general these regions comprise a promoter which, in prokaryotes, contains both the promoter per se, i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/- 10 boxes and the Shine-Dalgarno element in prokaryotes or the TATA box, CAAT sequences, and 5'- capping elements in eukaryotes. These regions can also include enhancer or repressor elements as well as translated signal and leader sequences for targeting the native polypeptide to a specific compartment of a host cell.

In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

In various embodiments, a vector comprising a nucleic acid molecule of the invention can therefore comprise a regulatory sequence, preferably a promoter sequence. In certain embodiments, the promoter is identical or homologous to promoter sequences of the host genome. In such cases endogenous polymerases may be capable to transcribe the nucleic acid molecule sequence comprised in the vector. In various embodiments, the promoter is selected from the group of weak, intermediate and strong promoters, preferably from weak to intermediate promoters.

In another preferred embodiment, a vector comprising a nucleic acid molecule of the present invention comprises a promoter sequence and a transcriptional termination sequence. Suitable promoters for prokaryotic expression are, for example, the araBAD promoter, the tet-promoter, the lacUV5 promoter, the CMV promo tor, the EF1 alpha promotor, the AOX1 promotor, the tac promotor, the T7promoter, or the lac promotor. Examples of promoters useful for expression in eukaryotic cells are the SV40 promoter or the CMV promoter. Furthermore, a nucleic acid molecule of the invention can comprise transcriptional regulatory elements, e.g., repressor elements, which allow regulated transcription and translation of coding sequences comprised in the nucleic acid molecule. Repressor element may be selected from the group consisting of the Lac-, AraC-, or MaIR-repressor.

The vector may be effective for prokaryotic or eukaryotic protein expression. Suitable vectors are known to those skilled in the art.

The vectors of the present invention may be chosen from the group consisting of high, medium and low copy vectors.

The above described vectors of the present invention may be used for the transformation or transfection of a host cell in order to achieve expression of a peptide or protein which is encoded by an above described nucleic acid molecule and comprised in the vector DNA. Thus, in a further aspect, the present invention also relates to a host cell comprising a vector or nucleic acid molecule as disclosed herein.

Also contemplated herein are host cells, which comprise a nucleic acid molecule as described herein integrated into their genomes. The skilled person is aware of suitable methods for achieving the nucleic acid molecule integration. For example, the molecule may be delivered into the host cells by means of liposome transfer or viral infection and afterwards the nucleic acid molecule may be integrated into the host genome by means of homologous recombination. In certain embodiments, the nucleic acid molecule is integrated at a site in the host genome, which mediates transcription of the peptide or protein of the invention encoded by the nucleic acid molecule. In various embodiments, the nucleic acid molecule further comprises elements which mediate transcription of the nucleic acid molecule once the molecule is integrated into the host genome and/or which serve as selection markers.

In certain embodiments, the nucleic acid molecule of the present invention is transcribed by a polymerase natively encoded in the host genome. In various embodiments, the nucleic acid molecule is transcribed by a RNA-polymerase which is non-native to the host genome. In such embodiments, the nucleic acid molecule of the present invention may further comprise a sequence encoding for a polymerase and/or the host genome may be engineered or the host cell may be infected to comprise a nucleic acid sequence encoding for an exogenous polymerase. The host cell may be specifically chosen as a host cell capable of expressing the gene. In addition or otherwise, in order to produce the isolated polypeptide of the invention, the nucleic acid coding for it can be genetically engineered for expression in a suitable system. Transformation can be performed using standard techniques (Sambrook, J. et al. (2001), Molecular Cloning: A Laboratory Manual, 3rd Ed., Cold Spring Harbor Laboratory Press, Cold Spring Harbor, NY).

Prokaryotic or eukaryotic host organisms comprising such a vector for recombinant expression of the polypeptide as described herein form also part of the present invention. Suitable host cells can be prokaryotic cells. In certain embodiments the host cells are selected from the group consisting of gram positive and gram negative bacteria. In some embodiments, the host cell is a gram negative bacterium, such as E.coli. In certain embodiments, the host cell is E. coli. In further embodiments, the host cell is selected from the group consisting of Escherichia coli (E. coli), Pseudomonas, Serratia marcescens, Salmonella, Shigella (and other enterobacteriaceae), Neisseria, Hemophilus, Klebsiella, Proteus, Enter obacter, Helicobacter, Acinetobacter, Moraxella, Helicobacter, Stenotrophomonas, Bdellovibrio, Legionella, acetic acid bacteria, Bacillus, Bacilli, Carynebacterium, Clostridium, Listeria, Streptococcus, Staphylococcus, and Archaea cells. Suitable eukaryotic host cells are among others CHO cells, insect cells, fungi, yeast cells, e.g., Saccharomyces cerevisiae, S. pombe, Pichia pastoris, and the like.

The transformed host cells are cultured under conditions suitable for expression of the nucleotide sequence encoding the polypeptide of the invention. In certain embodiments, the cells are cultured under conditions suitable for expression of the nucleotide sequence encoding a polypeptide of the invention and, optionally, its secretion.

For producing the recombinant polypeptide of interest described herein, a vector of the invention can be introduced into a suitable prokaryotic or eukaryotic host organism by means of recombinant DNA technology (as already outlined above). For this purpose, the host cell is first transformed with a vector comprising a nucleic acid molecule according to the present invention using established standard methods (Sambrook, J. et al. (2001), supra). The host cell is then cultured under conditions, which allow expression of the heterologous DNA and thus the synthesis of the corresponding polypeptide. Subsequently, the polypeptide is recovered either from the cell or from the cultivation medium.

For expression of the polypeptides of the present invention several suitable protocols are known to the skilled person. The expression of a recombinant polypeptide of the present invention may be achieved by the following method comprising: (a) introducing a nucleic acid molecule or vector of the invention into a host cell, wherein the nucleic acid molecule or vector encodes the recombinant polypeptide; and (b) cultivating the host cell in a culture medium under conditions that allow expression of the recombinant polypeptide.

Step (a) may be carried out by using suitable transformation and transfection techniques known to those skilled in the art. These techniques are usually selected based on the type of host cell into which the nucleic acid is to be introduced. In some embodiments, the transformation may be achieved using electroporation or heat shock treatment of the host cell.

Step (b) may include a cultivation step that allows growth of the host cells. Alternatively, such step allowing growth of the host cells and a step that allows expression of the polypeptide may be performed separately in that the cells are first cultivated such that they grow to a desired density and then they are cultivated under conditions that allow expression of the polypeptide. The expression step can however still allow growth of the cells.

The method may further include a step of recovering the expressed polypeptide. The polypeptide may be recovered from the growth medium, if it is secreted, or from the cells or both. The recovery of the polypeptide may include various purification steps.

Generally, any known culture medium suitable for growth of the selected host may be employed in this method.

In various embodiments, the method also encompasses the purification the recombinant polypeptide, wherein the recombinant polypeptide is purified using a method selected from affinity chromatography, ion exchange chromatography, reverse phase chromatography, size exclusion chromatography, and combinations thereof.

In several embodiments, the method may comprise the treatment of the recombinant polypeptide with a protease suitable for cleavage of a protease cleavage site within the recombinant polypeptide. In some embodiments, the recombinant polypeptide is purified prior to proteolytic cleavage using one or more methods disclosed above. Also after cleavage of the recombinant peptide or protein, the method may comprise a further purification step as defined above. Thus, in some embodiments the recombinant polypeptide is purified, subjected to proteolytic cleavage and the resulting polypeptide is further purified. In other embodiments, the protease may be co-expressed or added to the cultivation medium or expressed by co-cultivated microorganisms, such that cleavage occurs before purification.

In a further aspect, the present invention relates to the use of a vector or nucleic acid molecule as disclosed herein for the expression of a recombinant polypeptide. In some embodiments, the vector is used for the expression and optionally secretion of a recombinant polypeptide. The expression or expression and secretion may be achieved using the method described herein.

A method for expression of a recombinant polypeptide using the above-described nucleic acid molecules may comprise the steps of:
(a) introducing a nucleic acid molecule or a vector as described above into a suitable host cell, wherein the nucleic acid molecule or vector encodes the recombinant polypeptide; and
(b) cultivating the host cell in a culture medium under conditions that allow expression of the recombinant polypeptide and optionally secretion of the recombinant polypeptide into the culture medium.

In various embodiments, the invention also relates to a pharmaceutical composition comprising the recombinant IFNa2 polypeptide of the invention, the nucleic acid of claim of the invention or the vector of the invention and, optionally, at least one pharmaceutically acceptable excipient. In preferred embodiments, the pharmaceutical composition comprises the polypeptide of the invention. It may be provided in a form that allows administration to a subject in need thereof, such as, for example, an injectable solution. Generally, the pharmaceutical compositions may be suitable for parenteral (e.g. intravenous, intramuscular, intrathecal, and subcutaneous), topical, oral, inhalable, or local administration.

"Pharmaceutically acceptable excipients" are typically auxiliaries, diluents and carriers used in the pharmaceutical arts for formulation of an active. In case of injectable solutions, these may include various buffer compositions, salts, preservatives and the like. All of these are typically sterile. Examples, without limitation, include Ringer's solution, normal saline, sterile water, or any other commercially prepared physiological buffer solution designed for intravenous infusion. It will be understood that the selection of the carrier solution, and the dosage and administration of the composition, will vary with the subject and the particular clinical setting, and will be governed by standard medical procedures.

In accordance with the methods of the present invention, these pharmaceutical compositions may be administered in amounts effective to inhibit or ameliorate the pathological consequences or symptoms associated with the disease and disorders identified herein, in particular viral infections. Administration may be by bolus intravenous injection, by constant intravenous infusion, or by a combination of both routes. Alternatively, or in addition, the polypeptides mixed with appropriate excipients may be taken into the circulation via an intramuscular site.

Clearly, polypeptides are less suitable for oral, administration due to susceptibility to digestion by gastric acids or intestinal enzymes, however in certain embodiments specific formulations may be used for oral administration.

In preparing the compositions in oral liquid dosage forms (e.g., suspensions, elixirs and solutions), typical pharmaceutical media, such as water, glycols, oils, alcohols, flavoring agents, preservatives, coloring agents and the like can be employed.

Similarly, when preparing oral solid dosage forms (e.g., powders, tablets and capsules), carriers such as starches, sugars, diluents, granulating agents, lubricants, binders, disintegrating agents and the like will be employed. Due to their ease of administration, tablets and capsules represent a desirable oral dosage form for the pharmaceutical compositions of the present invention.

Polypeptides are less suitable for topical administration, however for local treatment specific formulations may be used.

The recombinant IFNa2 polypeptides and pharmaceutical compositions of the present invention are particularly useful for intravenous administration. The compositions for administration will commonly comprise a solution of the polypeptide dissolved in a pharmaceutical acceptable carrier, preferably in an aqueous carrier. A variety of aqueous carriers can be used, e.g., buffered saline and the like. These solutions are sterile and generally free of undesirable matter. The compositions may be sterilized by conventional, well-known sterilization techniques. A typical pharmaceutical composition for intravenous administration can be readily determined by one of ordinary skill in the art. The amounts administered are clearly protein specific and depend on its potency and pharmacokinetic profile. Actual methods for preparing parenterally administrable compositions will be known or apparent to those skilled in the art and are described in more detail in such publications as Remington's Pharmaceutical Science, 18t ed., Mack Publishing Company, Easton, PA, 1990.

The compositions containing the polypeptides of the invention or a cocktail thereof (i.e., with other proteins) can be administered in therapeutic treatments. In therapeutic applications, compositions are administered to a patient suffering from any of the disease/disorders identified herein in an amount sufficient to cure or at least partially alleviate symptoms the disorder. An amount adequate to accomplish this is defined as a "therapeutically effective dose". Amounts effective for this use will depend upon the severity of the disease and the general state of the patient's health.

Single or multiple administrations of the compositions may be administered depending on the dosage and frequency as required and tolerated by the patient. In any event, the composition should provide a sufficient quantity of the polypeptides of this invention to effectively treat the patient. Generally, depending on the intended mode of administration, the pharmaceutical acceptable compositions will contain about 1 % to about 99% by weight of a polypeptide of the invention, and 99% to 1 % by weight of a suitable pharmaceutical excipient or carrier. Preferably, the composition will be about 5% to 75% by weight of a polypeptide(s) of the invention, with the rest being suitable pharmaceutical excipients or carriers.

The polypeptides of the present invention, or their pharmaceutical acceptable compositions, are administered in a therapeutically effective amount, which will vary depending upon a variety of factors including the specific activity of the particular polypeptide employed; the metabolic stability and length of action of the polypeptide; the age, body weight, general health, sex, and diet of the patient; the mode and time of administration; the rate of excretion; the drug combination; the severity of the particular disease-states; and the host undergoing therapy. Generally, a therapeutically effective daily dose may range from about 0.1 µg to about 1000 µg/kg of body weight per administration of a polypeptide of the invention, preferably, from about 0.5 µg to about 100 µg/kg of body weight per administration. For example, for administration to a 70 kg person, the dosage range would be from about 10 µg to about 100 µg per administration of a polypeptide of the invention depending on the treatment regimen. For example, if the polypeptide of the invention or formulation containing the polypeptide is administered from one to several times a day, then a lower dose would be needed than if a formulation was administered weekly, or monthly or less frequently. It is anticipated that polypeptides of the present invention are administered in proper doses. Doses are generally adjusted to at or below the maximum tolerated dose (MTD).

The polypeptide or the pharmaceutical composition described herein may be for use in the treatment and/or prevention of a disease or disorder in in a subject in need thereof, in particular the treatment and/or prevention of an infection by a pathogen. Said disease or disorder may include cancer, immunological disorders, and infectious diseases, such as those caused by bacterial or viral pathogens or certain parasites. In various embodiments, the pathogen is a virus, for example, selected from the group consisting of influenza virus, corona virus, zika virus, dengue virus, west nile virus, ebola virus hepatitis B virus, hepatitis D virus, hepatitis A virus, hepatitis E virus, adenovirus, herpes viruses (CMV, EBV, HSV, KSHV, VZV), RSV, BKV, JCV, and HIV.

In various embodiments, the subject is a mammal, preferably a human being.

Further embodiments are described in the following non-limiting examples.

### EXAMPLES

### Materials and methods

### Expression protocol

### Cloning of IFNa2 variants

Site-directed mutations were introduced using sequence-specific primers and the QuikChangell kit (Agilent) according to manufacturer's instructions. All constructs were confirmed by sequence determination of the coding sequence.

### Expression and purification of IFNa2 variants

IFN alpha 2 variant genes were optimized for expression in *Escherichia coli.* Isolated inclusion body proteins denatured with guanidine hydrochloride were refolded in arginine refolding buffer and purified by anion-exchange and size exclusion chromatography (Kalie et al., J Biol Chem 282:11602-11611). Protein concentrations were determined using Nano-Drop 2000c (Thermo Scientific, Wilmington, DE), and endotoxin levels were less than 0.0025 endotoxin units (EU)/ml (ToxinSensor; Genscript, Piscataway, NJ).

### Characterization of IFNa2 variants

A stable reporter cell line using human retinal pigment epithelial cells (ATCC CRL2302) transfected with a plasmid containing interferon-stimulated response element (ISRE) promoter/enhancer elements driving a luciferase reporter gene (pISRE; Stratagene) (Zimmermann et al., J Exp Med 201:1543-1553) was developed. Cells were grown for 24 h before adding serial dilutions of our recombinant IFNa2 variants and different commercially available IFNa subtypes (PBL Assay Science, Piscataway, NJ) for 4 h. The cells were lysed with Bright Glo lysis buffer (Promega), and luciferase activity was measured 10 min later.

Primary human hepatocytes (PHH) were infected with HBV virions at 200 viral genome equivalents (vge) per cell in the presence of 4% PEG8000 for 16 hours and then washed by PBS. The culture medium with or without IFNa subtypes or IFNa2 chimeric mutants was changed every 3 days. The levels of HBV e antigen (HBeAg) and HBV s antigen (HBsAg) in the cell culture supernatant were determined by ELISA (Kits from Kehua, China). The results are shown in Figure 1, 5 and 6.

### Generation of HBV-infected humanized mice and IFNα treatments

Fah/Rag2/II2rγ triple knockout (FRG KO) mice purchased from Yecuris Corporation were utilized in the establishment of human liver chimeric mice. Nitisinone (NTBC) is supplemented in the drinking water to substitute for loss of FAH enzyme in maintaining mouse liver function. Briefly, 1.0x10⁶ PHH (Lonza) were injected intra-splenically into 4-6 weeks old mice followed by a number of NTBC cycling withdrawal conditions to facilitate mouse hepatocytes damage which in turn allow human hepatocytes to repopulate. Human albumin (hALB) production was measured by ELISA (Bethyl Laboratories Inc.) whereby ∼5mg/ml represents ∼70% humanization of mouse liver at 2-3 months post-transplantation. Once ∼70% of humanized mouse liver is achieved, 1.0 x 10⁷ IU/ml of HBV (Genotype D, produced by HepAD38 cell lines) was injected by tail vein. HBV DNA (Qiagen) was measured in mice serum on a weekly basis. HBV-infected mice were randomized into the different treatment groups based on body weight, blood human albumin (h-Alb), and serum HBV DNA concentrations. PBS control and IFNα treatments were administered intravenously daily for 14 days from day 45 post-infection onwards. A series of serological and intrahepatic measurements with the samples collected at the indicated time points was carried out (Fig. 2). The IFN treatment were overall well tolerated by the mice, even though the treatment protocol involved frequent handling and blood draws and daily injection of IFNs or saline (Fig. 3, 4). Both IFNα2 and -a14 treatment interfered with the HBV DNA and HBsAg increase and significantly reduced the HBV RNA production (Fig. 2). All mice were sacrificed at 10 weeks post HBV-infection.

### Antiviral efficacy of IFNa subtypes against influenza virus infection

1000 U/mL of IFNα subtypes were added to the supernatants of lung explants. 200 µL of the drug-containing medium were injected into the tissue, followed by incubation for a total of 8 h at 37°C and 5%CO₂. IFN and virus-containing supernatants were removed and viral replication was analyzed by titrating virus titers in the supernatants 1, 24 and 48 hours post infection by plaque assay (Fig. 7).

All documents cited herein, are hereby incorporated by reference in their entirety. The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. Recombinant interferon alpha 2 (IFNa2) polypeptide comprising an amino acid sequence having at least 80 %, at least 85 %, at least 90 %, at least 95%, at least 97.5%, at least 99% or at least 99.5% sequence identity over its entire length with the amino acid sequence set forth in SEQ ID NO:1 and comprising one or more amino acid substitution(s) in any one or more of the positions corresponding to positions 2, 10, 11, 14, 15, 16, 22, 26, 34, 37, 42, 51, 52, 54, 55, 56, 59, 60, 63, 74, 79, 82, 84, 86, 89, 92, 100, 102, 104, 106, 107, 108, 109, 112, 120, 131, 132, 153, 155, 159, 160, 163, and 165 of SEQ ID NO:1, or fragments thereof.

2. The recombinant interferon alpha 2 (IFNa2) polypeptide of claim 1, wherein the polypeptide comprises amino acid substitution(s) in
(i) any one or more of the positions corresponding to positions 2, 10, 26, 37, 51, 52, 54, 60, 63, 82, 86, 89, 92, 120, 131, and 153 of SEQ ID NO:1;
(ii) any one or more of the positions corresponding to positions 11, 14, 16, 22, 26, 34, 42, 51, 52, 54, 55, 56, 63, 86, 89, 100, 102, 106, 108, 109, 112, 131, 132, 153, 159, 160, 163, and 165 of SEQ ID NO:1;
(iii) any one or more of the positions corresponding to positions 11, 15, 37, 52, 54, 59, 63, 74, 79, 84, 100, 102, 104, 106, 107, 112, 131, and 153 of SEQ ID NO:1;
(iv) the positions corresponding to positions 82, 86, 89, and 120 of SEQ ID NO:1;
(v) the positions corresponding to 26 and 153 of SEQ ID NO:1;
(vi) the positions corresponding to positions 26, 82, 86, 89, 120, and 153 of SEQ ID NO:1;
(vii) the positions corresponding to positions 51, 52, 54, 60, and 63 of SEQ ID NO:1;
(viii) the positions corresponding to positions 2, 10, 37, 92, and 131 of SEQ ID NO:1;
(ix) the positions corresponding to positions 86 and 89 of SEQ ID NO:1;
(x) the positions corresponding to positions 16, 22, 26, and 153 of SEQ ID NO:1;
(xi) the positions corresponding to positions 16, 22, 26, 86, 89, and 153 of SEQ ID NO:1;
(xii) the positions corresponding to positions 52, 54, 55, 56, and 63 of SEQ ID NO:1;
(xiii) the positions corresponding to positions 11, 14, 34, 42, 51, 100, 102, 106, 108, 109, 112, 131, 132, 159, 160, 163, and 165 of SEQ ID NO:1;
(xiv) the positions corresponding to positions 15 and 153 of SEQ ID NO:1;
(xv) the positions corresponding to positions 52, 54, 59, and 63 of SEQ ID NO:1; or
(xvi) the positions corresponding to positions 11, 37, 74, 79, 84, 100, 102, 104, 106, 107, 112, 131 and 155, 160, 163 of SEQ ID NO:1.

3. The recombinant interferon alpha 2 (IFNa2) polypeptide of claim 1 or 2, wherein the amino acid substitution(s) are selected from the group consisting of: 2N, 10N, 11N, 11H, 14A, 15M, 161, 22G, 26P, 26Y, 34N, 37E, 37R, 42V, 51Q, 52A, 54S, 55A, 56F, 59V, 60M, 63T, 74V, 79R, 82E, 84L, 86I, 89F, 92M, 100T, 100M, 102E, 104W, 106G, 106E, 1081, 107G, 109A, 112N, 120K, 131M, 131T, 132G, 153F, 153S, 155R, 159K, 160G, 160R, 163R, and 165D.

4. The recombinant interferon alpha 2 (IFNa2) polypeptide of any one of claims 1-3, wherein the polypeptide comprises, using the numbering of SEQ ID NO:1,
(i) any one or more amino acid substitution(s) selected from the group consisting of 2N, 10N, 26P, 37E, 51Q, 52A, 54S, 60M, 63T, 82E, 86I, 89F, 92M, 120K, 131M, and 153F;
(ii) any one or more amino acid substitution(s) selected from the group consisting of 11N, 14A, 161, 22G, 26Y, 34N, 42V, 51Q, 52A, 54S, 55A, 56F, 63T, 86I, 89F, 100T, 102E, 106E, 1081, 109A, 112N, 131M, 132G, 153F, 159K, 160G, 163R, and 165D;
(iii) any one or more of the positions corresponding to positions 11H, 15M, 37R, 52A, 54S, 59V, 63T, 74V, 79R, 84L, 100M, 102E, 104W, 106G, 107G, 112N, 131T, and 153S of SEQ ID NO:1;
(iv) the amino acid substitution(s) 82E, 86I, 89F, and 120K;
(v) the amino acid substitution(s) 26P and 153F;
(vi) the amino acid substitution(s) 26P, 82E, 86I, 89F, 120K, and 153F;
(vii) the amino acid substitution(s) 51Q, 52A, 54S, 60M, and 63T;
(viii) the amino acid substitution(s) 2N, 10N, 37E, 92M, and 131M;
(ix) the amino acid substitution(s) 86I and 89F;
(x) the amino acid substitution(s) 161, 22G, 26Y, and 153F;
(xi) the amino acid substitution(s) 161, 22G, 26Y, 861, 89F, and 153F;
(xii) the amino acid substitution(s) 52A, 54S, 55A, 56F, and 63T;
(xiii) the amino acid substitution(s) 11N, 14A, 34N, 42V, 51Q, 100T, 102E, 106E, 1081, 109A, 112N, 131M, 132G, 159K, 160G, 163R, and 165D;
(xiv) the positions corresponding to positions 15M and 153S of SEQ ID NO:1;
(xv) the positions corresponding to positions 52A, 54S, 59V, and 63T of SEQ ID NO:1; or
(xvi) the positions corresponding to positions 11H, 37R, 74V, 79R, 84L, 100M, 102E, 104W, 106G, 107G, 112N, 131T, 155R, 160R, and 163R of SEQ ID NO:1.

5. The recombinant interferon alpha 2 (IFNa2) polypeptide of any one of claims 1-4, wherein the polypeptide comprises, using the numbering of SEQ ID NO:1,
(A) any combination of two or more of the following sets of amino acid substitution(s)
(1) 82E, 86I, 89F, and 120K;
(2) 26P and 153F;
(3) 51Q, 52A, 54S, 60M, and 63T;
(4) 2N, 10N, 37E, 92M, and 131M;
(B) any combination of two or more of the following sets of amino acid substitution(s)
(1) 861 and 89F;
(2) 161, 22G, 26Y, and 153F;
(3) 52A, 54S, 55A, 56F, and 63T;
(4) 11N, 14A, 34N, 42V, 51Q, 100T, 102E, 106E, 1081, 109A, 112N, 131M, 132G, 159K, 160G, 163R, and 165D;
(C) any combination of two or more of the following sets of amino acid substitution(s)
(1) 15M and 153S;
(2) 52A, 54S, 59V, and 63T;
(3) 11H, 37R, 74V, 79R, 84L, 100M, 102E, 104W, 106G, 107G, 112N, 131T, 155R, 160R, and 163R.

6. The recombinant interferon alpha 2 (IFNa2) polypeptide of claim 5, wherein the polypeptide comprises, using the numbering of SEQ ID NO:1, the following combination of amino acid substitutions
(1) 82E, 86I, 89F, 120K; 26P and 153F;
(2) 82E, 86I, 89F, 120K, 51Q, 52A, 54S, 60M, and 63T;
(3) 82E, 86I, 89F, 120K, 2N, 10N, 37E, 92M, and 131M;
(4) 26P, 153F, 51Q, 52A, 54S, 60M, and 63T;
(5) 26P, 153F, 2N, 10N, 37E, 92M, and 131M;
(6) 51Q, 52A, 54S, 60M, 63T, 2N, 10N, 37E, 92M, and 131M;
(7) 82E, 86I, 89F, 120K, 51Q, 52A, 54S, 60M, 63T, 2N, 10N, 37E, 92M, and 131M;
(8) 26P, 153F, 51Q, 52A, 54S, 60M, 63T, 2N, 10N, 37E, 92M, and 131M;
(9) 82E, 86I, 89F, 120K, 26P, 153F, 51Q, 52A, 54S, 60M, 63T, 2N, 10N, 37E, 92M, and 131M;
(10) 86I, 89F, 161, 22G, 26Y, and 153F;
(11) 86I, 89F, 52A, 54S, 55A, 56F, and 63T;
(12) 86I, 89F, 11N, 14A, 34N, 42V, 51Q, 100T, 102E, 106E, 1081, 109A, 112N, 131M, 132G, 159K, 160G, 163R, and 165D;
(13) 161, 22G, 26Y, 153F, 52A, 54S, 55A, 56F, and 63T;
(14) 16I, 22G, 26Y, 153F, 11N, 14A, 34N, 42V, 51Q, 100T, 102E, 106E, 1081, 109A, 112N, 131M, 132G, 159K, 160G, 163R, and 165D;
(15) 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(16) 86I, 89F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(17) 16I, 22G, 26Y, 153F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(18) 86I, 89F, 161, 22G, 26Y, 153F, 52A, 54S, 55A, 56F, 63T, 11N, 14A, 34N, 42V, 51Q, 100T, 102E, 106E, 1081, 109A, 112N, 131M, 132G, 159K, 160G, 163R, and 165D;
(19) 15M, 52A, 54S, 59V, 63T, and 153S;
(20) 11H, 15M, 37R, 74V, 79R, 84L, 100M, 102E, 104W, 106G, 107G, 112N, 131T, and 153S;
(21) 11H, 37R, 52A, 54S, 59V, 63T, 74V, 79R, 84L, 100M, 102E, 104W, 106G, 107G, 112N, 131T, 155R, 160R, and 163R; or
(22) 11H, 15M, 37R, 52A, 54S, 59V, 63T, 74V, 79R, 84L, 100M, 102E, 104W, 106G, 107G, 112N, 131T, 153S, 155R, 160R, and 163R.

7. The recombinant interferon alpha 2 (IFNa2) polypeptide of any one of claims 1-6, wherein the fragment
(i) is at least 100 amino acids in length, preferably at least 110, at least 120, at least 130, at least 140, at least 150, at least 160, 161, 162, 163 or 164 amino acids in length; and/or
(ii) retains at least one of the substitutions.

8. The recombinant interferon alpha 2 (IFNa2) polypeptide of any one of claims 1-7, wherein the polypeptide is conjugated to a second moiety, preferably a PEG moiety.

9. Nucleic acid encoding the polypeptide of any one of claims 1 to 8.

10. Vector comprising a nucleic acid molecule according to claim 9.

11. Host cell comprising a nucleic acid molecule according to claim 9 or a vector according to claim 10.

12. Method for the production of a polypeptide of any one of claims 1 to 7, comprising
(1) cultivating the host cell of claim 10 under conditions that allow the expression of the polypeptide; and
(2) isolating the expressed polypeptide from the host cell.

13. Pharmaceutical composition comprising the polypeptide of any one of claims 1 to 8, the nucleic acid of claim 9 or the vector of claim 10 and, optionally, at least one pharmaceutically acceptable excipient.

14. The polypeptide of any one of claims 1 to 8 or the pharmaceutical composition of claim 13 for use in the treatment of an infection by a pathogen in a subject in need thereof.

15. The polypeptide or the pharmaceutical composition for use according to claim 14, wherein (1) the pathogen is a virus or bacteria, preferably a virus selected from the group consisting of influenza virus, corona virus, zika virus, dengue virus, west nile virus, ebola virus hepatitis B virus, hepatitis D virus, hepatitis A virus, hepatitis E virus, adenovirus, herpes viruses (CMV, EBV, HSV, KSHV, VZV), RSV, BKV, JCV, and HIV. (2) the subject is a human.


SEQUENCE LISTING

<110>  Universitaet Duisburg-Essen

<120>  INTERFERON ALPHA 2 VARIANTS AND USES THEREOF

<130>  P77790

<160>  14

<170>  PatentIn version 3.5

<210>  1
<211>  165
<212>  PRT
<213>  Homo sapiens

<400>  1

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165

<210>  2
<211>  166
<212>  PRT
<213>  Homo sapiens

<400>  2

Cys Asn Leu Ser Gln Thr His Ser Leu Asn Asn Arg Arg Thr Leu Met
1               5                   10                  15

Leu Met Ala Gln Met Arg Arg Ile Ser Pro Phe Ser Cys Leu Lys Asp
            20                  25                  30

Arg His Asp Phe Glu Phe Pro Gln Glu Glu Phe Asp Gly Asn Gln Phe
        35                  40                  45

Gln Lys Ala Gln Ala Ile Ser Val Leu His Glu Met Met Gln Gln Thr
    50                  55                  60

Phe Asn Leu Phe Ser Thr Lys Asn Ser Ser Ala Ala Trp Asp Glu Thr
65                  70                  75                  80

Leu Leu Glu Lys Phe Tyr Ile Glu Leu Phe Gln Gln Met Asn Asp Leu
                85                  90                  95

Glu Ala Cys Val Ile Gln Glu Val Gly Val Glu Glu Thr Pro Leu Met
            100                 105                 110

Asn Glu Asp Ser Ile Leu Ala Val Lys Lys Tyr Phe Gln Arg Ile Thr
        115                 120                 125

Leu Tyr Leu Met Glu Lys Lys Tyr Ser Pro Cys Ala Trp Glu Val Val
    130                 135                 140

Arg Ala Glu Ile Met Arg Ser Leu Ser Phe Ser Thr Asn Leu Gln Lys
145                 150                 155                 160

Arg Leu Arg Arg Lys Asp
                165


<210>  3
<211>  166
<212>  PRT
<213>  Homo sapiens

<400>  3

Cys Asp Leu Pro Gln Thr His Ser Leu Gly Asn Arg Arg Ala Leu Ile
1               5                   10                  15

Leu Leu Ala Gln Met Gly Arg Ile Ser His Phe Ser Cys Leu Lys Asp

20                  25                  30

Arg Tyr Asp Phe Gly Phe Pro Gln Glu Val Phe Asp Gly Asn Gln Phe
        35                  40                  45

Gln Lys Ala Gln Ala Ile Ser Ala Phe His Glu Met Ile Gln Gln Thr
    50                  55                  60

Phe Asn Leu Phe Ser Thr Lys Asp Ser Ser Ala Ala Trp Asp Glu Thr
65                  70                  75                  80

Leu Leu Asp Lys Phe Tyr Ile Glu Leu Phe Gln Gln Leu Asn Asp Leu
                85                  90                  95

Glu Ala Cys Val Thr Gln Glu Val Gly Val Glu Glu Ile Ala Leu Met
            100                 105                 110

Asn Glu Asp Ser Ile Leu Ala Val Arg Lys Tyr Phe Gln Arg Ile Thr
        115                 120                 125

Leu Tyr Leu Met Gly Lys Lys Tyr Ser Pro Cys Ala Trp Glu Val Val
    130                 135                 140

Arg Ala Glu Ile Met Arg Ser Phe Ser Phe Ser Thr Asn Leu Gln Lys
145                 150                 155                 160

Gly Leu Arg Arg Lys Asp
                165

<210>  4
<211>  165
<212>  PRT
<213>  Artificial

<220>
<223>  IFNa2/IFNa14 chimeric mutant

<400>  4

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Glu Lys Phe Tyr Ile Glu Leu Phe Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Lys Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165

<210>  5
<211>  165
<212>  PRT
<213>  Artificial

<220>
<223>  IFNa2/IFNa14 chimeric mutant

<400>  5

Cys Asp Leu Pro Gln Thr His Ser Leu Gly Ser Arg Arg Thr Leu Met
1               5                   10                  15

Leu Leu Ala Gln Met Arg Lys Ile Ser Pro Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Phe Ser Thr Asn Leu Gln Glu Ser
145                 150                 155                 160

Leu Arg Ser Lys Glu
                165

<210>  6
<211>  165
<212>  PRT
<213>  Artificial

<220>
<223>  IFNa2/IFNa14 chimeric mutant

<400>  6

Cys Asp Leu Pro Gln Thr His Ser Leu Gly Ser Arg Arg Thr Leu Met
1               5                   10                  15

Leu Leu Ala Gln Met Arg Lys Ile Ser Pro Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Glu Lys Phe Tyr Ile Glu Leu Phe Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Lys Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Phe Ser Thr Asn Leu Gln Glu Ser
145                 150                 155                 160

Leu Arg Ser Lys Glu
                165

<210>  7
<211>  165
<212>  PRT
<213>  Artificial

<220>
<223>  IFNa2/IFNa14 chimeric mutant

<400>  7

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Ala Ile Ile Ser Val Leu His Glu Met Met Gln Gln Thr Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu

                165

<210> 8
<211> 165
<212> PRT
<213> Artificial

<220>
<223> IFNa2/IFNa14 chimeric mutant

<400> 8

Cys Asn Leu Pro Gln Thr His Ser Leu Asn Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Glu Phe Pro Gln Glu Glu Phe Gly Asn Gln Phe Gln
        35                  40                  45

Lys Ala Glu Thr Ile Pro Val Leu His Glu Met Ile Gln Met Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Met Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Met Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165

<210> 9
<211> 165
<212> PRT
<213> Artificial

<220>

<223>  IFNa2/IFNa16 chimeric mutant

<400>  9

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Ile Glu Leu Phe Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165


<210>  10
<211>  165
<212>  PRT
<213>  Artificial

<220>
<223>  IFNa2/IFNa16 chimeric mutant

<400>  10

Cys Asp Leu Pro Gln Thr His Ser Leu Gly Ser Arg Arg Thr Leu Ile
1               5                   10                  15

Leu Leu Ala Gln Met Gly Lys Ile Ser Tyr Phe Ser Cys Leu Lys Asp

20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Phe Ser Thr Asn Leu Gln Glu Ser
145                 150                 155                 160

Leu Arg Ser Lys Glu
                165

<210>  11
<211>  165
<212>  PRT
<213>  Artificial

<220>
<223>  IFNa2/IFNa16 chimeric mutant

<400>  11

Cys Asp Leu Pro Gln Thr His Ser Leu Gly Ser Arg Arg Thr Leu Ile
1               5                   10                  15

Leu Leu Ala Gln Met Gly Lys Ile Ser Tyr Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Ile Glu Leu Phe Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Phe Ser Thr Asn Leu Gln Glu Ser
145                 150                 155                 160

Leu Arg Ser Lys Glu
                165

<210>  12
<211>  165
<212>  PRT
<213>  Artificial

<220>
<223>  IFNa2/IFNa16 chimeric mutant

<400>  12

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Ala Ile Ser Ala Phe His Glu Met Ile Gln Gln Thr Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Thr Pro Leu Met Lys
            100                 105                 110

Glu Asp Ser Ile Leu Ala Val Arg Lys Tyr Phe Gln Arg Ile Thr Leu
        115                 120                 125

Tyr Leu Lys Glu Lys Lys Tyr Ser Pro Cys Ala Trp Glu Val Val Arg
    130                 135                 140

Ala Glu Ile Met Arg Ser Phe Ser Leu Ser Thr Asn Leu Gln Glu Ser
145                 150                 155                 160

Leu Arg Ser Lys Glu
                165

<210>  13
<211>  165
<212>  PRT
<213>  Artificial

<220>
<223>  IFNa2/IFNa16 chimeric mutant

<400>  13

Cys Asp Leu Pro Gln Thr His Ser Leu Gly Asn Arg Arg Ala Leu Met
1               5                   10                  15

Leu Leu Ala Gln Met Arg Lys Ile Ser Leu Phe Ser Cys Leu Lys Asp
            20                  25                  30

Arg Asn Asp Phe Gly Phe Pro Gln Glu Val Phe Gly Asn Gln Phe Gln
        35                  40                  45

Lys Ala Gln Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Thr Gln Glu Val Gly Val Glu Glu Ile Ala Leu Met Asn
            100                 105                 110

Glu Asp Ser Ile Leu Ala Val Arg Lys Tyr Phe Gln Arg Ile Thr Leu
        115                 120                 125

Tyr Leu Met Gly Lys Lys Tyr Ser Pro Cys Ala Trp Glu Val Val Arg
    130                 135                 140

Ala Glu Ile Met Arg Ser Phe Ser Leu Ser Thr Asn Leu Gln Lys Gly
145                 150                 155                 160

Leu Arg Arg Lys Asp
                165

<210>  14
<211>  166
<212>  PRT
<213>  Homo sapiens

<400>  14

Cys Asp Leu Pro Gln Thr His Ser Leu Gly His Arg Arg Thr Met Met
1               5                   10                  15

Leu Leu Ala Gln Met Arg Arg Ile Ser Leu Phe Ser Cys Leu Lys Asp
            20                  25                  30

Arg His Asp Phe Arg Phe Pro Gln Glu Glu Phe Asp Gly Asn Gln Phe
        35                  40                  45

Gln Lys Ala Glu Ala Ile Ser Val Leu His Glu Val Ile Gln Gln Thr
    50                  55                  60

Phe Asn Leu Phe Ser Thr Lys Asp Ser Ser Val Ala Trp Asp Glu Arg
65                  70                  75                  80

Leu Leu Asp Lys Leu Tyr Thr Glu Leu Tyr Gln Gln Leu Asn Asp Leu
                85                  90                  95

Glu Ala Cys Val Met Gln Glu Val Trp Val Gly Gly Thr Pro Leu Met
            100                 105                 110

Asn Glu Asp Ser Ile Leu Ala Val Arg Lys Tyr Phe Gln Arg Ile Thr
        115                 120                 125

Leu Tyr Leu Thr Glu Lys Lys Tyr Ser Pro Cys Ala Trp Glu Val Val
    130                 135                 140

Arg Ala Glu Ile Met Arg Ser Phe Ser Ser Ser Arg Asn Leu Gln Glu
145                 150                 155                 160

Arg Leu Arg Arg Lys Glu
                165